# EUROPÄISCHE PATENTSCHRIFT

(11) **EP 0 777 739 B1**
(45) Veröffentlichungstag und Bekanntmachung des Hinweises auf die Patenterteilung: **05.04.2000**
(21) Anmeldenummer: 95930524.4
(22) Anmeldetag: 25.08.1995
(51) Int. Cl.: C12N 15/85, A61K 48/00, C12N 15/86

(54) **GENTHERAPEUTISCHE BEHANDLUNG VON GEFÄSSERKRANKUNGEN DURCH EINEN ZELLSPEZIFISCHEN, ZELLZYKLUSABHÄNGIGEN WIRKSTOFF**
GENETIC THERAPY OF VASCULAR DISEASES WITH A CELL-SPECIFIC ACTIVE SUBSTANCE WHICH IS DEPENDENT ON THE CELL CYCLE
THERAPIE GENETIQUE DE MALADIES VASCULAIRES AVEC UNE SUBSTANCE ACTIVE SPECIFIQUE DE LA CELLULE ET DEPENDANT DU CYCLE CELLULAIRE

(30) Priorität: 26.08.1994 GB 9417366; 29.03.1995 GB 9506466
(43) Veröffentlichungstag der Anmeldung: 11.06.1997
(73) Patentinhaber: HOECHST AKTIENGESELLSCHAFT, 65926 Frankfurt am Main (DE)
(72) Erfinder: SEDLACEK, Hans-Harald, D-35041 Marburg (DE); MÜLLER, Rolf, D-35037 Marburg (DE)
(74) Vertreter: Dost, Wolfgang, Dr.rer.nat., Dipl.-Chem.
(86) Internationale Anmeldenummer: EP9503368
(87) Internationale Veröffentlichungsnummer: WO9606938

(56) Entgegenhaltungen:
- WO-A-93/10135
- WO-A-93/13807
- WO-A-94/29469
- BRITISH JOURNAL OF HAEMATOLOGY, Bd. 87, Nr. Supplement 1, 2. - 5.Juni 1994 Seite 124 F.EHLERT ET AL. 'Cell cycle-regulated transcription of the human cdc25C gene is controlled by a novel regulatory element'

## Beschreibung

### Technisches Gebiet

Es wird eine DNA-Sequenz für die Gentherapie von Gefäßerkrankungen beschrieben. Wesentliche Elemente für die DNA-Sequenz sind die Aktivatorsequenz, das Promotormodul und das Gen für die Wirksubstanz. Die Aktivatorsequenz wird zellspezifisch aktiviert in glatten Muskelzellen, aktivierten Endothelzellen, aktivierten Makrophagen oder aktivierten Lymphozyten. Diese Aktivierung wird zellzyklusspezifisch reguliert durch das Promotormodul. Die Wirksubstanz stellt einen Inhibitor für das Wachstum glatter Muskelzellen und/ oder für die Gerinnung dar. Die beschriebene DNA-Sequenz wird eingefügt in einen viralen oder nicht-viralen Vektor, ergänzt um einen Liganden mit Affinität für die Zielzelle.

### 1) Gefäßerkrankungen durch glatte Muskelzellen

Glatte Muskelzellen der Gefäße sind vorwiegend in der arteriellen Tunica media lokalisiert und an der lokalen und systemischen Blutdruckregelung beteiligt. Beim unverletzten, gesunden Gefäß sind diese glatten Muskelzellen im Ruhezustand der Zellteilung (R. Ross, Nature 362, 801 (1993)). Verletzungen der Gefäße führen zur Wanderung von glatten Muskelzellen in die Intimaschicht der Gefäßwand, wo sie proliferieren (Neointimabildung) und extrazelluläre Matrixkomponenten bilden.

Die intimale Proliferation von glatten Muskelzellen wird als eine wesentliche Komponente bei der Entstehung der Arteriosklerose angesehen (J.S. Forrester et al., Am. Coll. Cardiol. 17, 758 (1991)). Des weiteren führt diese Proliferation glatter Muskelzellen zur Restenose der Gefäße nach angioplastischen Operationen wie auch nach der Ballondilatation von eingeengten Gefäßen (R.S. Schwartz et al., Am. Coll. Cardiol. 20, 1284 (1992), M.W. Liu et al., Circulation 79, 1374 (1989)).

Bekanntermaßen führt die Arteriosklerose wie auch die Stenose und Restenose von Gefäßen zur schlußendlichen Thrombose des Gefäßes und damit häufig zu einem lebensbedrohlichen Infarkt.

Bislang ist jedoch noch keine Therapie verfügbar, welche durch Hemmung des Wachstums von glatten Muskelzellen Stenosen von Gefäßen verhindert. Zwar ist bekannt, daß Heparin die Proliferation glatter Muskelzellen zu hemmen vermag (Cochran et al., J. Cell Physiol. 124, 29 (1995), mit Heparin konnte jedoch die Entstehung von Stenosen nicht ausreichend verhindert werden. Es war somit naheliegend, daß neue Verfahren geprüft wurden, das Wachstum von glatten Muskelzellen in den verletzten Gefäßen zu verhindern und hierdurch das Infarktrisiko zu beheben. Hierbei wurde die Kenntnis von den Genen und Molekülen genutzt, welche in das Wachstum von glatten Muskelzellen regulierend eingreifen.

So ist bekannt, daß das Protoonkogen c-myb wie auch die cdc2-Kinase und das "proliferating cell nuclear antigen (PCNA)" an der Proliferation von glatten Muskelzellen beteiligt sind. Durch Verabreichung von Antisense c-myb Oligonukleotiden (Simons et al., Nature 359, 67 (1992)) wie auch von Antisense cdc2-Kinase Oligonukleotiden in Kombination mit Antisense PCNA Oligonukleotiden (Morishita et al., Proc. Natl. Acad. Sci. 90, 8474 (1993)) unmittelbar nach und lokal am Ort der Gefäßschädigung konnte eine Proliferation von glatten Muskelzellen in der Ratte verhindert werden.

Ähnliche Ergebnisse wurden in der Ratte und beim Schwein erzielt mit der Verabreichung des als Onkogensuppressor bekannten Retinoblastoma(Rb)genes, auch hier unmittelbar nach und lokal am Ort der Gefäßschädigung gegeben. Um eine Inaktivierung des Rb-Genproduktes durch Phosphorylierung zu verhindern, wurde ein punktmutiertes Rb-Gen verwendet (Austausch von Thr-246, Ser-601, Ser-605, Ser-780, Ser-786, Ser-787, Ser-800 mit Ala, von Thr-350 mit Arg und von Ser-804 mit Glu (Hamel et al., Mol. Cell Biol. 12, 3431 (1992)), welches eine nicht phosphorylierbare, konstitutiv aktive Form des Rb-Proteins kodiert. Dieses mutierte Rb-Gen wurde eingebaut in einen replikationsdefizienten rekombinanten Adenovirus und dieser Vektor lokal verabreicht (Chang et al., Science 267, 518 (1995)).

In einem anderen Versuchsansatz wurde ein replikationsdefizientes rekombinantes Adenovirus verwendet, in welchem das Gen für die Herpes simplex-Virus-Thymidinkinase eingefügt war (AV-HS-TK). Das Kinase-Gen-Produkt ist in der Lage, die Wirkstoffvorstufe ("Prodrug") Ganciclovir zu phosphorylieren und hierdurch in ein Nukleosidanalog umzuwandeln, welches die DNA-Synthese inhibiert.

Der Genvektor AV-HS-TK wurde 7 Tage nach der Gefäßschädigung, jedoch auch hier lokal am Ort der Schädigung verabreicht und nachfolgend täglich über 14 Tage Ganciclovir intraperitoneal injiziert. Durch diese Behandlung wurde bei der Ratte eine deutliche Hemmung des Wachstums der glatten Muskelzellen erzielt (Guzman et al., Proc. Natl. Acad. Sci. 91, 10732 (1994)). Mit einer gleichartigen Behandlung wurden ähnliche Ergebnisse auch am Schwein erzielt (Ohno et al., Science 265, 781 (1994)). Die Gabe des Vektors erfolgte hier jedoch unmittelbar nach der Gefäßverletzung und die Ganciclovir-Verabreichung täglich über 6 Tage.

Insgesamt verdeutlichen diese Versuche die Möglichkeit, durch unterschiedliche gentherapeutische Maßnahmen, welche in die Zellteilungsvorgänge von glatten Muskelzellen eingreifen, die Stenose nach Verletzungen der Gefäße zu verhindern.

Nachteil der literaturbekannten Verfahren ist jedoch, daß die wirksamen Substanzen (Vektoren) lokal am Ort der Gefäßverletzungen appliziert werden müssen, wobei gegebenenfalls sogar der betreffende Gefäßabschnitt zeitweise verschlossen sein muß, um ein Abschwemmen der Vektoren zu verhindern. Derartige invasive Eingriffe werden im Rahmen der Ballondilatation von stenosierten Gefäßen zwar routinemäßig durchgeführt, benötigen jedoch einen beträchtlichen Aufwand und stellen ihrerseits eine erhebliche Gefährdung des Patienten durch das Risiko von Thrombosen und Embolien dar.

Zum anderen ist trotz der lokalen Applikation der Vektoren nicht gesichert, daß sie nur glatte, proliferierende Muskelzellen transduzieren. Die Transduktion anderer nahe oder entfernt gelegener Zellen könnte zu Nebenwirkungen (u.a. Transformation von Zellen und Tumorinduktionen) führen, wie sie heute in der Fachwelt diskutiert werden (Friedmann, Science 244, 1275 (1989), Plummer, Scrip Magazine III/29 (1995)).

Als Alternative zur Gabe der oben beschriebenen Vektoren ist die systemische (z.B. intravenöse oder orale) Gabe von Zytostatika für die Proliferationshemmung von glatten Muskelzellen am Ort der Gefäßerkrankung nur von geringer und vorübergehender Wirkung, weist andererseits das Risiko einer Schädigung von Endothelien auf und führt zu erheblichen akuten wie auch chronischen Nebenwirkungen.

### 2) Thrombosen

Thrombosen stellen eine immer noch schwer zu behandelnde, zum Teil lebensbedrohende Komplikation von metabolischen Erkrankungen, wie Arteriosklerose, arteriellen und venösen Gefäßerkrankungen und lokalen wie auch systemischen immunreaktiven Syndromen dar (Übersichten bei Philipps et al., Blood 71, 831 (1988), Harker, Biomed. Progr. 8, 17 (1995)).

Obwohl eine Reihe von Antikoagulantien, Antithrombotika, Fibrinolytika und Thrombozytenaggregationsinhibitoren bereits seit längerem in klinischer Anwendung und neue Substanzen in klinischer Erprobung sind, lassen sich die lebensbedrohenden Komplikationen der Thrombose bislang weder in ausreichendem Maße verhüten noch beherrschen (White, Scrip Magazine 4, 6 (1994), Antiplatelet Trialists' Collaboration BMJ 308, 81 (1994)).

Es besteht somit ein großer Bedarf nach neuen Arzneimitteln zur Verhütung und zur Therapie von Thrombosen (BMJ 305, 567 (1992), Vinazzer, Biomedical Progress 6, 17 (1993)). Ein beträchtlicher Teil der Thrombosen hat seine Ursache in aktivierten oder geschädigten Endothelzellen. Diese selbst oder die durch Wachstumsfaktoren im Blut zur Proliferation stimulierten glatten Muskelzellen bewirken alleine oder in Gesellschaft mit aktivierten Makrophagen, Lymphozyten, Thrombozyten und Granulozyten eine Aktivierung des Gerinnungssystems (Nemerson, Blood 71, 1 (1988)).

Diese Aktivierung führt letztendlich zur Bildung von Fibrin, zur Aktivierung und Aggregation von Thrombozyten und zur Bildung von Fibrin-reichen oder Thrombozyten-reichen blutgefäßverengenden oder verschließenden Blutgerinnseln, den Thrombosen. Derartige Thrombosen führen im Bereich des arteriellen Gefäßsystems zu Infarkten, welche beispielsweise am Herzen oder im Gehirn lebensbedrohlich sind.

Die bislang etablierte Therapie mit Antithrombotika (wie Heparin oder Fraktionen von Heparin), Antikoagulantien (wie Coumarin), Thrombozytenaggregationsinhibitoren (wie Aspirin) und Fibrinolytika (wie Streptokinase, Urokinase oder Gewebeplasminogenaktivatoren (tPA)) bewirkt zwar eine durch zahlreiche klinische Studien belegte prophylaktische Wirkung auf drohende Thrombosen und therapeutische Wirkung auf bestehende Thrombosen, diese Wirkung ist jedoch unzulänglich. Der Grund hierfür liegt zu einem beträchtlichen Teil in der Tatsache, daß die Wirkung der verwendeten Therapeutika nicht beschränkt ist auf den Ort der Erkrankung, d.h. der Thrombose, sondern daß sie systemisch wirken. Die hierdurch bedingten Blutungen beschränken somit sowohl die Dosiserhöhung als auch die Zeitdauer der Anwendung.

### 3) Allgemeine Beschreibung der Erfindung

Gegenstand der Erfindung ist nunmehr ein Wirkstoff (d. h. ein Arzneimittel), welcher lokal wie auch systemisch Patienten gegeben werden kann und welcher
- weitgehend nur glatte, in Zellteilung befindliche Muskelzellen beeinflußt, die Proliferation von glatten Muskelzellen nach Gefäßverletzungen oder Gefäßschäden hemmt und damit die Stenose oder Restenose von Gefäßen verhindert oder
- weitgehend nur am Ort einer entstehenden Thrombose, d.h. am Ort von aktivierten und proliferierenden Endothelzellen, glatten Muskelzellen der Intima, Makrophagen und/oder Lymphozyten die Blutgerinnung inhibiert oder
- welcher sowohl die Proliferation glatter Muskelzellen hemmt als auch dort die Thrombose lokal inhibiert.

Zentraler Bestandteil dieses Wirkstoffes ist ein DNA-Konstrukt, welches aus folgenden Elementen besteht:
(DNA wird im gesamten Text dieser Anmeldung als gemeinsamer Begriff sowohl für eine komplementäre (cDNA) als auch für eine genomische DNA-Sequenz benutzt).

Aktivatorsequenz
(UAS)
zellzyklusreguliertes
Promotormodul
Wirksubstanz

Das zentrale Element dieses Wirkstoffes stellt das zellzyklusregulierte Promotormodul dar.

Als zellzyklusreguliertes Promotormodul ist beispielsweise die Nukleotidsequenz - CDE-CHR-Inr- (siehe unten) zu verstehen. Die wesentliche Funktion des Promotormoduls ist die Hemmung der Funktion der Aktivatorsequenz in der G0/G1 Phase des Zellzyklus und eine Zellzyklus-spezifische Expression in der S/G2 Phase und damit in proliferierenden Zellen.

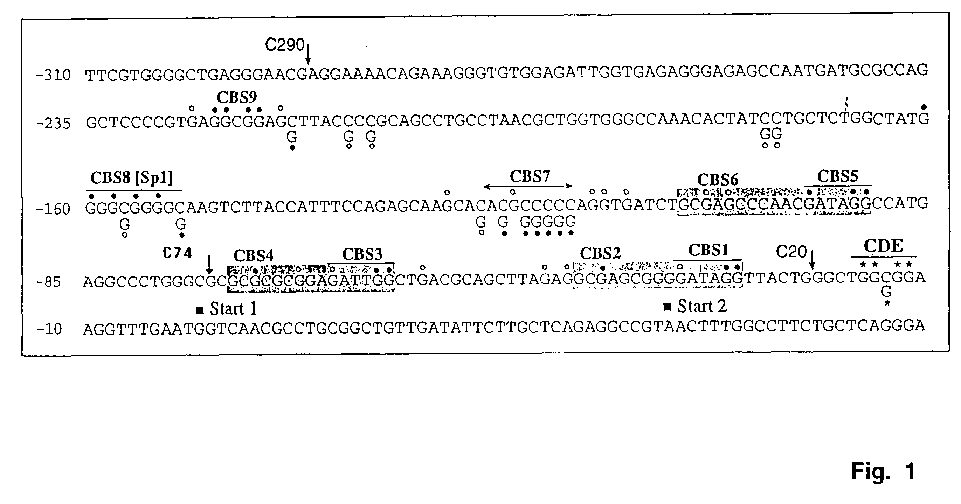

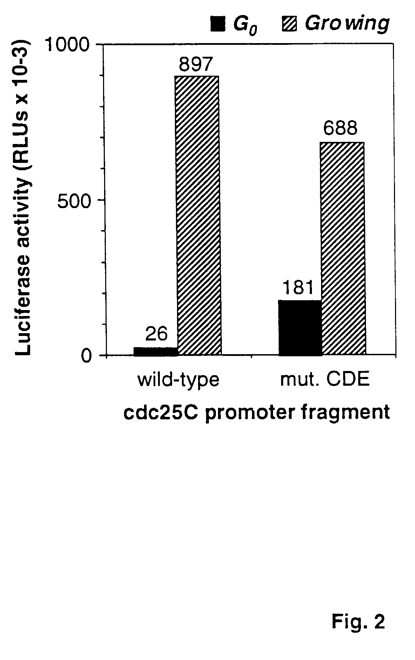

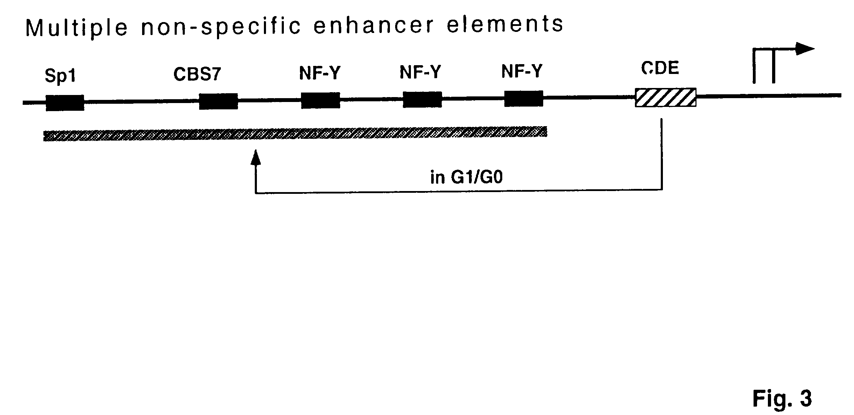

Das Promotormodul CDE-CHR-Inr wurde im Rahmen einer detaillierten Untersuchung der G2-spezifischen Expression des menschlichen cdc25C Promotors entdeckt. Ausgangspunkt war die Auffindung eines Repressorelementes ("cell cycle dependent element"; CDE), das für die Abschaltung des Promotors in der G1-Phase des Zellzyklus verantwortlich ist (Lucibello et al., EMBO J. 14, 132 (1995)). Durch genomisches Dimethylsulfat (DMS)-Footprinting und funktionelle Analysen (Fig. 1, 2) konnte gezeigt werden, daß das CDE einen Repressor ("CDE-binding factor"; CDF) G1-spezifisch bindet und hierdurch zu einer Inhibition der Transkription in nicht-proliferierenden (GO) Zellen führt. Das im Bereich des Basalpromotors lokalisierte CDE ist in seiner reprimierenden Funktion abhängig von einer "upstream activating sequence" (UAS). Dies führte zu dem Schluß, daß der CDE-bindende Faktor die transkriptionsaktivierende Wirkung 5' gebundener Aktivatorproteine in einer zellzyklusabhängigen Weise, d.h. in nicht-proliferierenden Zellen sowie in der G1-Phase des Zellzyklus, hemmt (Fig. 3).

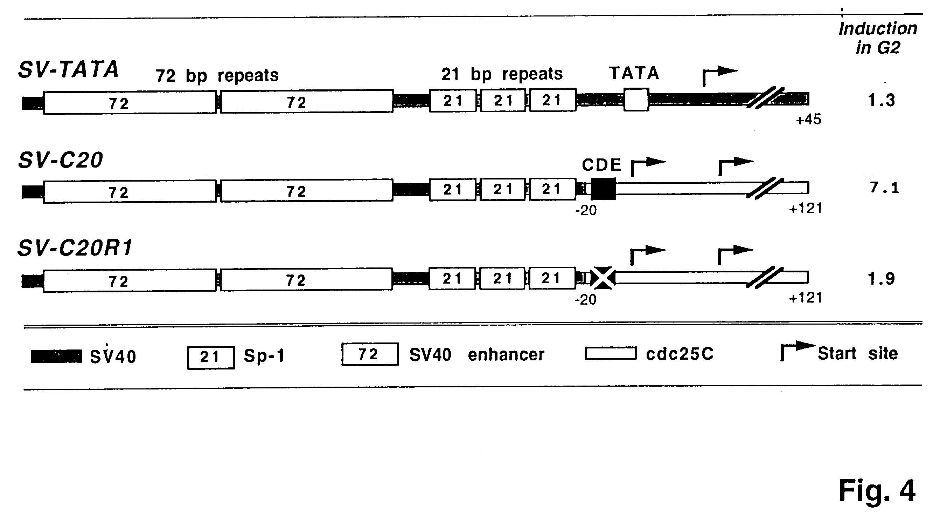

Diese Schlußfolgerung konnte durch ein weiteres Experiment bestätigt werden: Die Fusion des viralen, nicht-zellzyklusregulierten frühen SV40-Enhancers mit einem cdc25 Minimalpromotor (bestehend aus CDE und den 3' gelegenen Startstellen) führte zu einer klaren Zellzyklusregulation des chimären Promotors (Fig. 4). Nachfolgende Untersuchungen des cdc25C Enhancers haben gezeigt, daß es sich bei den vom CDF zellzyklusabhängig regulierten Transkriptionfaktoren um NF-Y (CBF) (Dorn et al., Cell 50, 863 (1987), Van Hujisduijnen et al., EMBO J. 9, 3119 (1990), Coustry et al., J. Biol. chem. 270, 468 (1995)), Sp1 ( Kadonaga et al., TIBS 11, 10 (1986)) und einen möglicherweise neuen an CBS7 bindenden Transkriptionsfaktor (CIF) handelt. Ein weiterer interessanter Befund dieser Studie war die Beobachtung, daß NF-Y innerhalb des cdc25C Enhancers nur in Kooperation mit mindestens einem weiteren NF-Y Komplex oder mit CIF effizient die Transkription aktiviert. Sowohl NF-Y als auch Sp1 gehören zur Klasse der glutaminreichen Aktivatoren, was wichtige Hinweise auf den Mechanismus der Repression (z.B. Interaktion bzw. Interferenz mit bestimmten basalen Transkriptionsfaktoren oder TAFs) liefert.

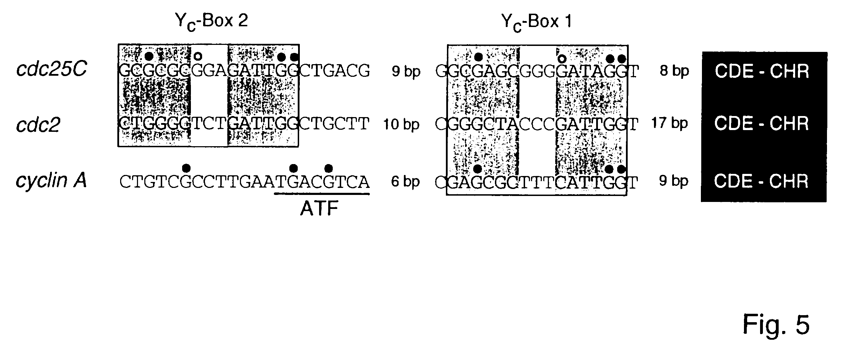

Ein Vergleich der Promotorsequenzen von cdc25C, cyclin A und cdc2 zeigte Homologien in mehreren Bereichen (Fig. 5). Nicht nur das CDE ist in allen 3 Promotoren konserviert (die vorhandenen Abweichungen sind funktionell nicht relevant), sondern auch die benachbarten Y_{C} -Boxen. Alle diese Bereiche zeigten wie erwartet Proteinbindung in vivo, im Falle des CDE in zellzyklusabhängiger Weise. Außerdem konnte gezeigt werden, daß alle 3 Promotoren durch eine Mutation des CDE dereguliert werden (Tabelle 1). Eine bemerkenswerte Ähnlichkeit wurde bei dem Vergleich der cdc25C, cyclin A und cdc2

Sequenzen auch im Bereich unmittelbar 3' vom CDE ("cell cycle genes homology region"; CHR) deutlich (Fig. 5). Dieser Bereich ist funktionell ebenso bedeutsam wie das CDE (Tabelle 1), wird in den in vivo Dimethylsulfat (DMS)-Footprinting Experimenten jedoch nicht sichtbar. Eine mögliche Erklärung hierfür ist eine Interaktion des Faktors mit der kleinen Furche der DNA. Ergebnisse aus "electrophoretic mobility shift assay" (EMSA) Experimenten deuten darauf hin, daß CDE und CHR gemeinsam einen Proteinkomplex, den CDF, binden. Diese Beobachtungen weisen daraufhin, daß die CDF-vermittelte Repression glutaminreicher Aktivatoren ein häufig vorkommender Mechanismus zellzyklusregulierter Transkription ist.

Von Bedeutung für die Regulation des cdc25C Promotors ist jedoch nicht nur der CDE-CHR-Bereich, sondern auch eine der Initiationsstellen (Position +1) innerhalb der Nukleotidsequenz des basalen Promotors (Positionen ≤ -20 bis ≥ +30, siehe Fig. 1). Mutationen in diesem Bereich, welcher die in vitro Bindestelle für den Transkriptionsfaktor YY-1 (Seto und Shenk, Nature 354, 241 (1991), Usheva und Shenk, Cell 76, 1115 (1994)) einschließt, führen zu einer völligen Deregulation. In Anbetracht der Nähe des CDE-CHR zum basalen Promotor ist somit eine Interaktion des CDF mit dem basalen Transkriptionskomplex sehr wahrscheinlich.

Als Aktivatorsequenz (upstream activator sequence = UAS) ist eine Nukleotidsequenz (Promotor- oder Enhancersequenz) zu verstehen, mit der Transkriptionsfaktoren gebildet oder aktiv in der Zielzelle interagieren. Als Aktivatorsequenz kann der CMV-Enhancer, der CMV-Promotor (EP 073.177.B1), der SV40 Promotor oder jede andere, dem Fachmann bekannt Promotor- oder Enhancersequenz verwendet werden. Im Sinne dieser Erfindung zählen jedoch zu den bevorzugten Aktivatorsequenzen solche genregulatorischen Sequenzen bzw. Elemente aus Genen, die besonders in glatten Muskelzellen, in aktivierten Endothelzellen oder in aktivierten Makrophagen oder Lymphozyten gebildete Proteine kodieren.

Als Wirksubstanz ist die DNA-Sequenz für ein Protein zu verstehen, welches am Ort der Entstehung den therapeutischen Effekt - das heißt die Inhibition der Proliferation der glatten Muskelzellen, der Gerinnung oder (im Falle zweier Wirksubstanzen) beides - bewirken kann. Die Auswahl der Nukleotidsequenz für die Aktivatorsequenz und für die Wirksubstanz richtet sich nach der Zielzelle und der gewünschten Wirksubstanz.

Das erfindungsgemäße DNA-Konstrukt wird in einer dem Fachmann geläufigen Weise zu einem Vektor vervollständigt; so wird es beispielsweise in einen viralen Vektor eingefügt (siehe hierzu D. Jolly, Cancer Gene Therapy 1, 51 (1994)), oder aber zu einem Plasmid ergänzt. Virale Vektoren oder Plasmide können mit kolloidalen Dispersionen komplexiert werden, so beispielsweise mit Liposomen (Farhood et al., Annals of the New York Academy of Sciences 716, 23 (1994)) oder aber mit Polylysin-Ligand-Konjugaten (Curiel et al., Annals of the New York Academy of Sciences 716, 36 (1994)). Ebenso mit den gebräuchlichen Arzneimittelhilfsstoffen kann eine Arzneimittelzubereitung erfolgen.

Derartige virale oder nicht-virale Vektoren können durch einen Liganden ergänzt werden, welcher Bindungsaffinität für eine Membranstruktur auf der ausgewählten Zielzelle hat. Die Auswahl des Liganden richtet sich somit nach der Auswahl der Zielzelle (siehe S. 21, 4.4 ff und S. 34, 5.4ff). Der erfindungsgemäße Wirkstoff wird anhand folgender Beispiele näher erläutert:

### 4) Wirkstoff zur Inhibition der Proliferation glatter Muskelzellen

### 4.1. Auswahl der Aktivatorsequenz für glatte Muskelzellen

Als Aktivatorsequenzen im Sinne der Erfindung sind bevorzugt genregulatorische Sequenzen bzw. Elemente aus Genen zu verwenden, die, besonders in glatten Muskelzellen, gebildete Proteine kodieren. Diese Gene sind beispielsweise:
- Tropomyosin
   (Tsukahara et al., Nucleic Acid Res. 22, 2318 (1994), Novy et al., Cell Motility and the Cytosceleton 25, 267 (1993), Wilton et al., Cytogenetics and Cell Genetics 68, 122 (1995))
- α-Actin
   (Sartorelli et al., Gens and Developm. 4, 1811 (1990), Miwa et al., Nucleic Acids Res. 18, 4263 (1990))
- α-Myosin
   (Kelly et al., Can. J. Physiol. and Pharm. 72, 1351 (1994), Moussavi et al., Mol. Cell. Biochem. 128, 219 (1993)
- Rezeptoren für Wachstumsfaktoren wie beispielsweise PDGF, FGF
   (Rubin et al., Int. Congress Ser. 925, 131 (1990), Ross, Ann. Rev. Med. 38, 71 (1987)
- Rezeptoren für Acetylcholin
   (Dutton et al., PNAS USA 90, 2040 (1993), Durr et al., Eur. J. Biochem. 224, 353 (1994))
- Phosphofructokinase-A
   (Gekakis et al., Biochemistry 33 1771 (1994), Tsujino et al., J. Biol. Chem. 264, 15334 (1989), Castella-Escola et al., Gene 91, 225 (1990))
- Phosphoglyceratemutase
   (Nakatsuji et al., Mol. Cell Biol. 12, 4384 (1992))
- Troponin C
   (Lin et al., Mol. Cell Biol. 11, 267 (1991))
- Desmin
   (Li et al., J. Biol. Chem. 266, 6562 (1991), Neuromuscular Disorders 3, 423 (1993))
- Myogenin
   (Funk et al., PNAS USA 89, 9484 (1992), Olson, Symp. Soc. Exp. Biol. 46, 331 (1992), Zhon et al., Mol. Cell. Biol. 14, 6232 (1994), Atchley et al., PNAS USA 91, 11522 (1994))
- Rezeptoren für Endothelin A
   (Hosoda et al., J. Biol. Chem. 267, 18797 (1992), Oreilly et al., J. Cardiovasc. Pharm. 22, 18 (1993), Hayzer et al., Am. J. Med. Sci. 304, 231 (1992), Haendler et al., J. Cardiovasc. Pharm. 20, 1 (1992))
- VEGF
   VEGF wird von glatten Muskelzellen, besonders unter hypoxischen Bedingungen, gebildet (Berse et al., Mol. Biol. Cell 3, 211 (1992), Finkenzeller et al., BBRC 208, 432 (1995), Tischer et al., BBRC 165, 1198 (1989), Leung et al., Science 246, 1306 (1989), Ferrara et al., Endoc. Rev. 13, 18 (1992)).
   Die Promotorsequenzen der Gene für diese Proteine sind durch folgende Arbeiten zugänglich:
- Tropomyosin
   (Gooding et al., Embo J. 13, 3861 (1994))
- α-Actin
   (Shimizu et al., J. Biol. Chem. 270, 7631 (1995), Sartorelli et al., Genes Dev. 4, 1811 (1999))
- α-Myosin
   (Kurabayashi et al., J. Biol. Chem. 265, 19271 (1990), Molkentin et al., Mol. Cell. Biol. 14, 4947 (1994))
- Rezeptor für PDGF
   (Pistritto et al., Antibiot. Chemother. 46, 73 (1994))
- Rezeptor für FGF
   (Myers et al., J. Biol. Chem. 270, 8257 (1995), Johnson et al., Adv. Cancer Res. 60, 1 (1993), Chellaiah et al., J. Biol. Chem. 269, 11620 (1994), Yu et al., Hum. Mol. Genetics 3, 212 (1994), Wang et al., BBRC 203, 1781 (1994), Murgue et al., Cancer Res. 54, 5206 (1994), Avraham et al., Genomics 21, 656 (1994), Burgess et al., Ann. Rev. Biochem. 58, 575 (1989))
- MRF-4
   (Naidu et al., Mol. Cell. Biol. 15, 2707 (1995))
- Phosphofructokinase A
   (Gekakis et al., Biochem. 33, 1771 (1994))
- Phosphoglyceratemutase
   (Makatsuji et al., Mol. Cell. Biol. 12, 4384 (1992))
- Troponin C
   (Ip et al., Mol. Cell. Biol. 14, 7517 (1994), Parmacek et al., Mol. Cell. Biol. 12, 1967 (1992))
- Myogenin
   (Salminen et al., J. Cell Biol. 115, 905 (1991), Durr et al., Eur. J. Biochem. 224, 353 (1994), Edmondson et al., Mol. Cell. Biol. 12, 3665 (1992))
- Rezeptoren für Endothelin A
   (Hosoda et al., J. Biol. Chem. 267, 18797 (1992), Li and Paulia, J. Biol. Chem. 266, 6562 (1991))
- Desmin
   (Li et al., Neuromusc. Dis. 3, 423 (1993), Li and Capetanaki, Nucl. Acids Res. 21, 335 (1993)).
- VEGF
   Die genregulatorischen Sequenzen für das VEGF-Gen sind
   * die Promotorsequenz des VEGF-Gens (5' flankierende Region)
      (Michenko et al., Cell Mol Biol. Res. 40, 35 (1994), Tischer et al., J. Biol. Chem. 266, 11947 (1991)) oder
   * die Enhancersequenz des VEGF-Gens (3' flankierende Region)
      (Michenko et al., Cell Mol. Biol. Res. 40, 35 (1994) oder
   * das c-Src-Gen
      (Mukhopadhyay et al., Nature 375, 577 (1995), Bonham et al., Oncogene 8, 1973 (1993), Parker et al., Mol. Cell. Biol. 5, 831 (1985), Anderson et al., Mol. Cell. Biol. 5, 1122 (1985)) oder
   * das V-Src-Gen
      (Mukhodpadhyay et al., Nature 375, 577 (1995), Anderson et al., Mol. Cell. Biol. 5, 1122 (1985), Gibbs et al., J. Virol. 53, 19 (1985))
   * "artifizielle" Promotoren
      Faktoren der Helix-Loop-Helix (HLH)-Familie (MyoD, Myf-5, Myogenin, MRF4 (Übersicht in Olson and Klein, Genes Dev. 8, 1 (1994)) sind als muskelspezifische Transkriptionsaktivatoren beschrieben. Des weiteren gehören zu den muskelspezifischen Transkriptionsaktivatoren das Zinkfingerprotein GATA-4 (Arceci et al., Mol Cell. Biol. 13, 2235 (1993); Ip et al., Mol. Cell. Biol. 14, 7517 (1994)) sowie die Gruppen der MEF-2 Transkriptionsfaktoren (Yu et al., Gene Dev. 6, 1783 (1992)).
   Die HLH-Proteine sowie GATA-4 zeigen muskelspezifische Transkription nicht nur mit Promotoren von muskelspezifischen Genen, sondern auch im heterologen Kontext, so auch mit artifiziellen Promotoren. Derartige artifizielle Promotoren sind beispielsweise:
   * multiple Kopien der (DNA) Bindestelle für muskelspezifische HLH-Proteine wie der E-Box (Myo D)
      (z.B. 4x AGCAGGTGTTGGGAGGC)
      (Weintraub et al., PNAS 87, 5623 (1990))
   * multiple Kopien der DNA Bindungsstelle für GATA-4 des ∝-Myosin-Heavy Chain Genes
      (z.B.5'-GGCCGATGGGCAGATAGAGGGGGCCGATGGGCAGATAGAGG-3')
      (Molkentin et al., Mol. Cell. Biol. 14, 4947 (1994))

### 4.2. Auswahl der Wirksubstanz für glatte Muskelzellen

Als Wirksubstanz in Sinne der Erfindung ist eine DNA-Sequenz zu verstehen, deren exprimiertes Protein die Proliferation von glatten Muskelzellen inhibiert. Zu diesen Zellzyklusinhibitoren gehören beispielsweise die DNA-Sequenzen für folgende Proteine:
a) inhibitorische Proteine
   - das Retinoblastomprotein (pRb=p110) oder die verwandten p107 und p130 Proteine (La Thangue, Curr. Opin. Cell Biol. 6, 443 (1994))
   - das p53 Protein (Prives et al., Genes Dev. 7, 529 (1993))
   - das p21 (WAF-1) Protein (El-Deiry et al., Cell 75, 817 (1993))
   - das p16 Protein (Serrano et al., Nature 366, 704 (1993), Kamb et al., Science 264, 436 (1994), Nobori et al., Nature 368, 753 (1994))
   - andere cdK-Inhibitoren (Übersicht bei Pines, TIBS 19, 143 (1995))
   - das GADD45 Protein (Papathanasiou et al., Mol. Cell. Biol. 11, 1009 (1991), Smith et al., Science 266, 1376 (1994))
   - das bak Protein (Farrow et al., Nature 374, 731 (1995), Chittenden et al., Nature 374, 733 (1995), Kiefer et al., Nature 374, 736 (1995)).

   Um eine schnelle intrazelluläre Inaktivierung dieser Zellzyklusinhibitoren zu verhindern, sind bevorzugt solche Gene zu verwenden, welche Mutationen für die Inaktivierungsstellen der exprimierten Proteine aufweisen, ohne daß diese hierdurch in ihrer Funktion beeinträchtigt werden.
   Das Retinoblastomprotein (pRb/p110) und die verwandten p107 und p130 Proteine werden durch Phosphorylierung inaktiviert. Bevorzugt wird somit eine pRb/p110 -, p107 - oder p130 cDNA-Sequenz verwendet, die derart mutiert ist, daß die Phosphorylierungsstellen des kodierten Proteins gegen nicht phosphorylierbare Aminosäuren ausgetauscht sind.
   Entsprechend Hamel et al. (Mol. Cell Biol. 12, 3431 (1992)wird die cDNA-Sequenz für das Retinoblastomprotein (p110) durch Austausch der Aminosäuren in den Positionen 246, 350, 601, 605, 780, 786, 787, 800 und 804 nicht mehr phosphorylierbar, seine Bindungsaktivität mit dem großen T-Antigen wird jedoch nicht beeinträchtigt. Beispielsweise werden die Aminosäuren Thr-246, Ser-601, Ser-605, Ser-780, Ser-786, Ser-787 und Ser-800 mit Ala, die Aminosäure Thr-350 mit Arg und die Aminosäure Ser-804 mit Glu ausgetauscht.
   In analoger Weise wird die DNA-Sequenz für das p107 Protein oder das p130 Protein mutiert.
   Das Protein p53 wird in der Zelle inaktiviert entweder durch Bindung an spezielle Proteine, wie beispielsweise MDM2 oder durch Oligomerisierung des p53 über das dephosphorylierte C-terminale Serin 392 (Schikawa et al., Leukemia und Lymphoma 11, 21 (1993) und Brown, Annals of Oncology 4, 623 (1993)). Bevorzugt wird somit eine DNA-Sequenz für ein p53 Protein verwendet, welches C-terminal verkürzt ist um das Serin 392.
b) zytostatische oder zytotoxische Proteine
   Als Wirksubstanz ist des weiteren eine DNA-Sequenz zu verstehen, die ein zytostatisches oder zytotoxisches Protein exprimiert.
   Zu derartigen Proteinen zählen beispielsweise
   - Perforin (Lin et al., Immunol. Today 16, 194 (1995))
   - Granzym (Smyth et al., Immunol. Today 16, 202 (1995))
   - TNF (Porter, TibTech 9, 158 (1991), Sidhu et al., Pharmac. Ther. 57, 79 (1993)), im speziellen
      * TNFα (Beutler et al., Nature 320, 584 (1986), Kriegler et al., Cell 53, 45 (1988)
      * TNFβ (Gray et al., Nature 312, 721 (1984), Li et al., J. Immunol. 138, 4496 (1987), Aggarwal et al., J. Biol. Chem. 260, 2334 (1985)
c) Enzyme
   Als Wirksubstanz ist jedoch auch die DNA-Sequenz für ein Enzym zu verstehen, welches eine inaktive Vorstufe eines Zytostatikums in ein Zytostatikum umwandelt.
   Derartige Enzyme, welche inaktive Vorsubstanzen (Prodrugs) in aktive Zytostatika (Drugs) spalten und die jeweils zugehörigen Prodrugs und Drugs sind bereits von Deonarain et al. (Br. J. Cancer 70, 786 (1994), von Mullen, Pharmac. Ther. 63, 199 (1994) und Harris et al., Gene Ther. 1, 170 (1994)) übersichtlich beschrieben worden.
   Beispielsweise ist die DNA-Sequenz eines der folgenden Enzyme zu verwenden:
   - Herpes Simplex Virus Thymidinkinase
      (Garapin et al., PNAS USA 76, 3755 (1979), Vile et al., Cancer Res. 53, 3860 (1993), Wagner et al., PNAS USA 78, 1441 (1981), Moelten et al., Cancer Res. 46, 5276 (1986), J. Natl. Cancer Inst. 82, 297 (1990))
   - Varizella Zoster Virus Thymidinkinase
      (Huber et al., PNAS USA 88, 8039 (1991), Snoeck, Int. J. Antimicrob. Agents 4, 211 (1994))
   - bakterielle Nitroreduktase
      (Michael et al., FEMS Microbiol. Letters 124, 195 (1994), Bryant et al., J. Biol. Chem. 266, 4126 (1991), Watanabe et al., Nucleic Acids Res. 18, 1059 (1990))
   - bakterielle β-Glucuronidase
      (Jefferson et al., PNAS USA 83, 8447 (1986)
   - pflanzliche β-Glucuroniase aus Secale cereale
      (Schulz et al., Phytochemistry 26, 933 (1987))
   - humane β-Glucuroniase
      (Bosslet et al., Br. J. Cancer 65, 234 (1992), Oshima et al., PNAS USA 84, 685 (1987))
   - humane Carboxy peptidase (CB) z.B.
      * CB-A der Mastzelle
         (Reynolds et al., J. Clin. Invest. 89, 273 (1992))
      * CB-B des Pankreas
         (Yamamoto et al., J. Biol. Chem. 267, 2575 (1992), Catasus et al., J. Biol. Chem. 270, 6651 (1995))
   - bakterielle Carboxy peptidase
      (Hamilton et al., J. Bacteriol. 174, 1626 (1992), Osterman et al., J. Protein Chem. 11, 561 (1992))
   - bakterielle β-Laktamase
      (Rodrigues et al., Cancer Res. 55, 63 (1995), Hussain et al., J. Bacteriol. 164, 223 (1985), Coque et al., Embo J. 12, 631 (1993)
   - bakterielle Cytosine deaminase
      (Mullen et al., PNAS USA 89, 33 (1992), Austin et al., Mol. Pharmac. 43, 380 (1993), Danielson et al., Mol. Microbiol. 6, 1335 (1992)
   - humane Catalase bzw. Peroxidase
      (Ezurum et al., Nucl. Acids Res. 21, 1607 (1993))
   - Phosphatase, im besonderen
      * humane alkalische Phosphatase
         (Gum et al., Cancer Res. 50, 1085 (1990))
      * humane saure Prostataphosphatase
         (Sharieff et al., Am. J. Hum. Gen. 49, 412 (1991), Song et al., Gene 129, 291 (1993), Tailor et al., Nucl. Acids Res. 18, 4928 (1990))
      * Typ 5 saure Phosphatase
         (Gene 130, 201 (1993))
   - Oxidase, im besonderen
      * humane Lysyloxidase
         (Kimi et al., J. Biol. Chem. 270, 7176 (1995))
      * humane saure D-aminooxidase
         (Fukui et al., J. Biol. Chem. 267, 18631 (1992))
   - Peroxidase, im besonderen
      * humane Gluthation Peroxidase
         (Chada et al., Genomics 6, 268 (1990), Ishida et al., Nucl. Acids Res. 15, 10051 (1987))
      * humane Eosinophilen Peroxidase
         (Ten et al., J. Exp. Med. 169, 1757 (1989), Sahamaki et al., J. Biol. Chem. 264, 16828 (1989))
      * humane Schilddrüsen Peroxidase
         (Kimura, PNAS USA 84, 5555 (1987)).

   Zur Erleichterung der Sekretion der aufgeführten Enzyme kann die jeweils in der DNA-Sequenz enthaltende homologe Signalsequenz ersetzt werden durch eine heterologe, die extrazelluläre Ausschleusung verbessernde Signalsequenz.
   So kann beispielsweise die Signalsequenz der β-Glucuronidase (DNA Position ≤ 27 bis 93; Oshima et al., PNAS 84, 685 (1987)) ersetzt werden durch die Signalfrequenz für das humane Immunglobulin (DNA Position ≤ 63 bis ≥ 107; Riechmann et al., Nature 332, 323 (1988).
   Des weiteren sind bevorzugt DNAs solcher Enzyme zu wählen, welche durch Punktmutationen in einem geringeren Maße in Lysosomen gespeichert werden. Derartige Punktmutationen wurden beispielsweise für die β-Glucuronidase beschrieben (Shipley et al., J. Biol. Chem. 268, 12193 (1993)).

### 4.3. Kombination von gleichen oder unterschiedlichen Wirksubstanzen für glatte Muskelzellen

Gegenstand der Erfindung ist des weiteren ein Wirkstoff, in welchem eine Kombination der DNA-Sequenzen von mehreren gleichen Wirksubstanzen (A,A) oder unterschiedlichen Wirksubstanzen (A,B) vorliegt. Zur Expression von zwei DNA-Sequenzen ist vorzugsweise die cDNA einer "internal ribosome entry site" (IRES) als regulatorisches Element zwischengeschaltet. Derartige IRES wurden von Mountford und Smith (TIG 11, 179 (1995), Kaufman et al., Nucl. Acids Res. 19, 4485 (1991), Morgan et al., Nucl. Acids Res. 20, 1293 (1992) und Dirks et al., Gene 129, 247 (1993), Pelletier und Sonenberg, Nature 334, 320 (1988), Sugitomo et al., BioTechn. 12, 694 (1994) beschrieben.

So kann die cDNA der IRES-Sequenz des Poliovirus (Position ≤ 140 bis ≥ 630 des 5' UTR (Pelletier und Sonenberg, Nature 334, 320 (1988)) zur Verknüpfung der DNA der antithrombotischen Substanz A (am 3' Ende) und der DNA der antithrombotischen Substanz B (am 5' Terminus) verwendet werden.

Aktivator-
sequenz
(UAS)

zellzyklus-
reguliertes
Promotor-
modul

Wirksubstanz
(Zellzyklus-
inhibitor)
A

internal
ribosome
entry
site

Wirksubstanz
(Zellzyklus-
inhibitor)
A oder B

Ein derartiger Wirkstoff weist je nach Kombination additive oder synergistische Wirkung im Sinne der Erfindung auf.

Im Gefolge des intimalen Wachstums der glatten Muskelzellen wie auch durch deren Apoptose oder Nekrose als Ergebnis der Einwirkung des Zellzyklusinhibitors kann das Gerinnungssystem aktiviert werden und können Thrombosen auftreten. Derartige Thrombosen sind durch eine prophylaktische Gabe eines Gerinnungshemmers (Aspirin, Heparin oder eines anderen Antithrombotikums) zu verhindern. Die Verabreichung des Gerinnungshemmers erfolgt systemisch, d.h. oral oder parenteral.

Häufig verhindern jedoch die Nebenwirkungen des Gerinnungshemmers eine ausreichende Konzentration am Ort des intimalen Wachstums der glatten Muskelzelle.

Somit ist die Prophylaxe der Thrombose durch derartige Gerinnungshemmer unsicher (Pukac, Am. J. Pathol. 139, 1501 (1991)).

Es ist nun ein weiterer Gegenstand der Erfindung, daß der im Sinne der Erfindung beanspruchte Wirkstoff zusätzlich zu einer Wirksubstanz, die einen Zellzyklusinhibitor darstellt, als weiteres Element die DNA-Sequenz für eine Wirksubstanz, die einen Gerinnungshemmer darstellt, enthält.

Aktivator-
sequenz
(UAS)

zellzyklus-
reguliertes
Promotor-
modul

Wirksubstanz
(Zellzyklus-
inhibitor)

internal
ribosome
entry
site

Wirksubstanz
Gerinnungs-
hemmer)

Die Expression des Gerinnungshemmers wird in gleicher Weise wie die Expression des Zellzyklusinhibitors durch die Aktivatorsequenz und das zellzyklusregulierte Repressormodul gesteuert. Die gleichzeitige Expression sowohl des Zellzyklusinhibitors als auch des Gerinnungshemmers wird vorzugsweise durch ein "internal ribosome entry site" (IRES) Genelement geregelt.

Als Gerinnungshemmer sind Gene für beispielsweise Plasminogenaktivatoren (PA), so der Gewebe-PA (tPA) oder der Urokinase-ähnliche PA (uPA), Protein C, Antithrombin-III, Tissue Factor Pathway Inhibitor oder Hirudin einzusetzen. Die DNA-Sequenzen für diese Gerinnungshemmer wurden wie folgt beschrieben:
- Tissue Plasminogen Activator (tPA)
   (Sasaki et al., Nucl. Acids Res. 16, 5695 (1988), Pennica et al., Nature 301, 214 (1983), Wei et al., DNA 4, 76 (1985), Harris et al., Mol. Biol. Med. 3, 279 (1986))
- Urokinase-type Plasminogen Activator (uPA)
   (Miyake et al., J. Biochem. 104, 643 (1988), Nelles et al., J. Biol. Chem. 262, 5682 (1987))
- Hybride von tPA und uPA
   (Kalyan et al., Gene 68, 205 (1988), Devries et al., Biochem. 27, 2565 (1988))
- Protein C
   (Foster et al., PNAS 82, 4673 (1985))
- Hirudin
   (Maerki et al., Semin. Thromb. Hemostas. 17, 88 (1991), De Taxis du Poet et al., Blood Coag. Fibrin. 2, 113 (1991), Harvey et al., PNAS USA 83, 1084 (1986), Sachhieri et al., EP 0324 712 B1, EP 0142 860 B1)
- Serin Proteinase Inhibitoren (Serpine), wie beispielsweise
   * C-1S-Inhibitor
      (Bock et al., Biochem. 25, 4292 (1986), Davis et al., PNAS USA 83, 3161 (1986), Que, BBRC 137, 620 (1986), Rauth et al., Proteine Sequences and Data Analysis 1, 251 (1988), Carter et al., Eur. J. Biochem. 173, 163 (1988), Tosi et al., Gene 42, 265 (1986), Carter et al., Eur. J. Biochem. 197, 301 /1991), Eldering et al., J. Biol. Chem. 267, 7013 (1993))
   * α1-Antitrypsin
      (Tosi et al., Gene 42, 265 (1986), Graham et al., Hum. Genetics 85, 381 (1990), Hafeez et al., J. Clin. Invest. 89, 1214 (1992), Tikunova et al., Bioorganicheskaja Khimia 17, 1694 (1991), Kay et al., Human Gene Ther. 3, 641 (1992), Lemarchand et al., Molekulaiaruaia Biologica 27, 1014 (1993), Lambach et al., Human Mol. Gen. 2, 1001 (1993))
   * Antithrombin III
      (Stackhouse et al., J. Biol. Chem. 258, 703 (1983), Olds et al., Biochem. 32, 4216 (1993), Laue et al., Nucl. Acids Res. 22, 3556 (1994))
- Tissue Factor Pathway Inhibitor (TFPI)
   (Enjyoji et al., Genomics 17, 423 (1993), Wun et al., J. Biol. Chem. 263, 6001 (1988), Girard et al., Thromb. Res. 55, 37 (1989))

### 4.4. Auswahl des Liganden für glatte Muskelzellen (siehe S. 9, Zeilen 5-21 supra)

Als Ligand in kolloidalen Dispersionen, beispielsweise Polylysin-Ligand-Konjugaten, werden Substanzen bevorzugt, welche an die Oberfläche von glatten Muskelzellen binden. Hierzu gehören Antikörper oder Antikörperfragmente, gerichtet gegen Membranstrukturen von glatten Muskelzellen, wie beispielsweise
- der Antikörper 10F3
   (Printseva et al., Exp. Cell Res. 169, 85 (1987),American J. Path. 134, 305 (1989)) oder
- Antikörper gegen Actin
   (Desmonliere et al., Comptes Reudus des Seances de la Soc. de Biol et de ses Filiales 182, 391 (1988)) oder
- Antikörper gegen Angiotensin II-Rezeptoren
   (Butcher et al., BBRA 196, 1280 (1993)) oder
- Antikörper gegen Rezeptoren für Wachstumsfaktoren
   (Übersichten bei Mendelsohn, Prog. All. 45, 147 (1988), Sato et al., J. Nat. Canc. Inst. 81, 1600 (1989), Hynes et al., BBA 1198, 165 (1994))
   oder Antikörper gerichtet beispielsweise gegen
   * EGF-Rezeptoren
      (Fan et al., Cancer Res. 53, 4322 (1993), Bender et al., Cancer Res. 52, 121 (1992), Aboud-Pirak et al., J. Nat. Cancer Inst. 80, 1605 (1988), Sato et al., Mol. Biol. Med. 1, 511 (1983), Kawamoto et al., PNAS 80, 1337 (1983))
   * oder gegen PDGF-Rezeptoren (Yu et al., J. Biol. Chem. 269, 10668 (1994), Kelly et al., J. Biol. Chem. 266, 8987 (1991), Bowen-Pope et al., J. Biol. Chem. 257, 5161 (1982))
   * oder gegen FGF-Rezeptoren (Vanhalteswaran et al., J. Cell Biol. 115, 418 (1991), Zhan et al., J. Biol. Chem. 269, 20221 (1994))
   * oder Antikörper gegen Endothelin A-Rezeptoren.

Die murinen monoklonalen Antikörper sind bevorzugt in humanisierter Form einzusetzen. Die Humanisierung erfolgt in der von Winter et al. (Nature 349, 293 (1991) und Hoogenbooms et al. (Rev. Tr. Transfus. Hemobiol. 36, 19 (1993) dargestellten Weise. Antikörperfragmente werden entsprechend dem Stand der Technik hergestellt, beispielsweise in der von Winter et al., Nature 349, 293 (1991), Hoogenboom et al., Rev. Tr. Transfus. Hemobiol. 36, 19 (1993), Girol, Mol. Immunol. 68, 1379 (1991) oder Huston et al., Int. Rev. Immunol. 10, 195 (1993) beschriebenen Weise.

Zu den Liganden gehören des weiteren alle Wirksubstanzen, welche an Membranstrukturen oder Membranrezeptoren auf glatten Muskelzellen binden (Übersicht bei Pusztai et al., J. Pathol. 169, 191 (1993), Harris, Current Opin. Biotechnol. 2, 260 (1991)). Beispielsweise gehören hierzu Wachstumsfaktoren oder deren Fragmente bzw. Teilsequenzen von ihnen, die an Rezeptoren exprimiert durch glatte Muskelzellen binden wie beispielsweise
- PDGF
   (Westermark et al., Cancer Res. 51, 5087 (1991), Ponten et al., J. Invest. Dermatol. 102, 304 (1994)
- EGF
   (Modjtahedi et al., Int. J. Oncol. 4, 277 (1994), Carpenter et al., J. Biol. Chem. 265, 7709 (1990))
- TGFβ
   (Segarini, BBA 1155, 269 (1993))
- TGFα
   (Salomon et al., Cancer Cells 2, 389 (1990)
- FGF
   (Burgess et al., Annu. Rev. Biochem. 58, 575 (1989)
- Endothelin A
   (Oreilly et al., J. Cardiovasc. Pharm. 22, 18 (1993)).

### 4.5. Herstellung des Wirkstoffes für glatte Muskelzellen

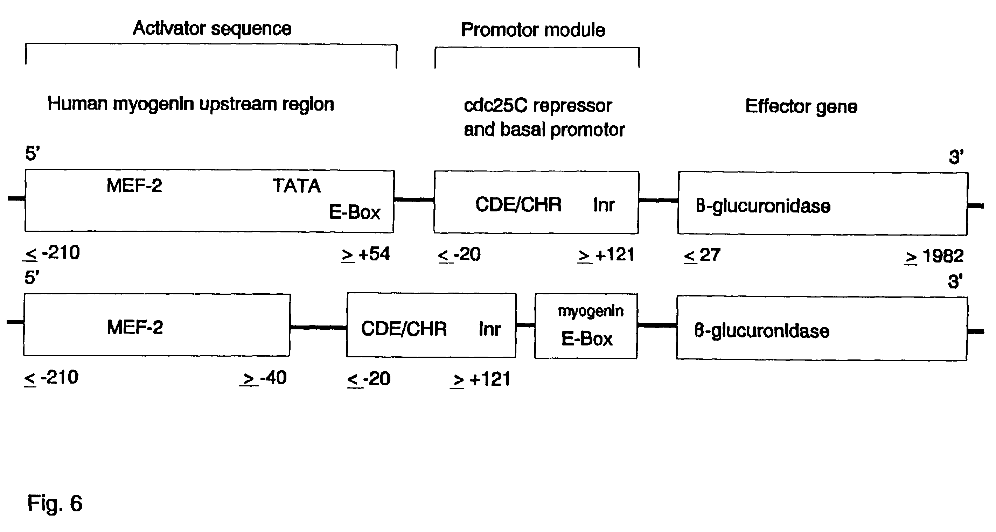

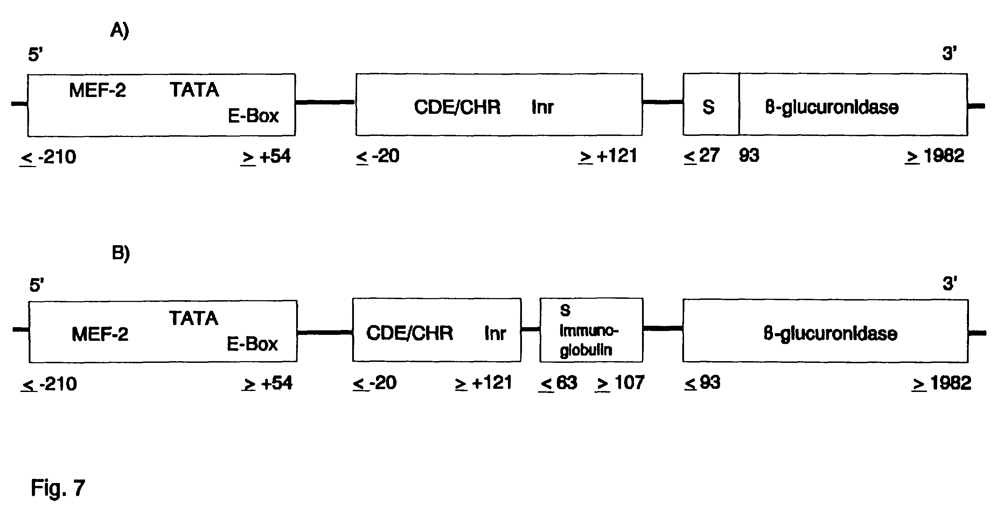

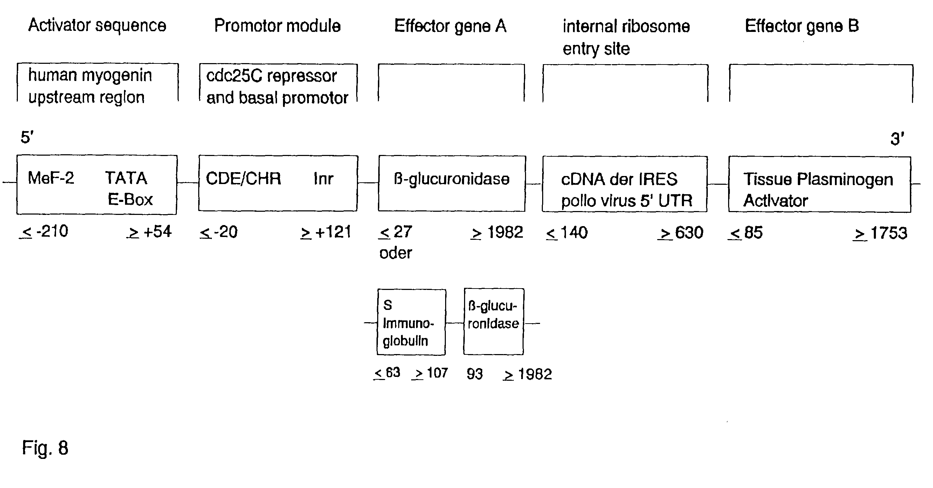

Die Herstellung des erfindungsgemäßen Wirkstoffes wird anhand folgender Beispiele näher beschrieben:
a) Konstruktion des chimären Promotors Myogenin-PromotorCDE-CHR-Inr
   Der humane Myogenin-Promotor (Pos. ≤ -210 bis ≥ +54, der von Salmin et al., J. Cell Biol. 115, 905 (1991) publizierten DNA-Sequenz) wird an seinem 3' Ende mit dem 5'-Terminus des CDE-CHR-Inr Moduls des humanen cdc25C-Gens (Pos. ≤ -20 bis ≥ +121, der von Lucibello et al., EMBO J. 14, 132 (1995) publizierten Sequenz) verknüpft (siehe Fig. 6). Die Verknüpfung erfolgt mit Hilfe von dem Fachmann bekannten und käuflichen Enzymen. Außerdem werden verschiedene Fragmente der Myogenin-Promotorsequenz verwendet (siehe Fig. 6). So wird die TATA-Box enthaltende DNA-Sequenz des Myogenin-Promotors verwendet. Jedoch kann gleichermaßen auch die Promotorsequenz Position ≤ -210 bis ≥ -40 zur Anwendung kommen (siehe Fig. 6).
b) Konstruktion eines Plasmids enthaltend den zentralen Bestandteil des Wirkstoffes
   Die so hergestellte chimäre Myogenin-Promotormodul-Transkriptionskontrolleinheit wird an ihrem 3' Ende mit dem 5'-Terminus einer DNA, die den kompletten kodierenden Bereich der humanen β-Glucuronidase (DNA Pos. ≤ 27- ≥ 1982, der von Oshima et al., PNAS USA 84, 684 (1987) publizierten Sequenz) enthält, verknüpft (siehe Fig. 6).
   Diese DNA enthält auch die für eine Sekretion notwendige Signalsequenz (22 N-terminale Aminosäuren). Zur Erleichterung der zellulären Ausschleusung ist diese Signalsequenz bevorzugt auszutauschen gegen die Signalsequenz des Immunglobulins (Position ≤ 63 bis ≥ 107; Riechmann et al., Nature 332, 323 (1988) (Fig. 7). Die so hergestellten Transkriptionskontrolleinheiten und die DNA für die humane β-Glucuronidase werden in pUC18/19 oder Bluescript-abgeleiteten Plasmidvektoren einkloniert, die direkt oder in kolloidalen Dispersionssystemen für eine in vivo Applikation genutzt werden können. Alternativ können die chimären Gene in virale Vektoren oder andere geeignete Vektoren transferiert und injuziert werden.
c) Konstruktion eines Plasmids enthaltend die Gene für β-Glucuronidase wie auch für Tissue Plasminogen Activator
   Das wie unter 2) hergestellte Myogenin-Repressormodul-β-Glucuronidase-Element wird an seinem 3' Ende mit dem 5'-Terminus der cDNA der "internal ribosome entry site" des Poliovirus (Position ≤ 140 bis ≥ 630 des 5' UTR Elementes, Pelletier und Sonenberg, Nature 334, 320 (1988)) verknüpft. An dessen 3' Ende wird wiederum der 5'-Terminus der DNA des Tissue Plasminogen Activator (Position ≤ 85 bis ≥ 1753, Pennica et al., Nature 301, 214 (1983)) verknüpft (Fig. 7). Anschließend wird das gesamte Konstrukt in pUC17/19 oder Bluescript-abgeleiteten Plasmidvektoren einkloniert, die direkt oder in kolloidalen Dispersionssystemen für einen in vivo Transfer genutzt werden können. Alternativ können die chimären Gene in virale Vektoren oder andere geeignete Vektoren transferiert werden (Fig. 8).

### 5. Wirkstoff zur Inhibition der Gerinnung

### 5.1. Auswahl der Aktivatorsequenz zur Inhibition oder Gerinnung

Als Aktivatorsequenzen im Sinne der Erfindung sind bevorzugt genregulatorische Sequenzen bzw. Elemente aus Genen zu verwenden, welche in glatten Muskelzellen, in aktivierten Endothelzellen, in aktivierten Makrophagen oder in aktivierten Lymphozyten nachweisbare Proteine kodieren.
a) glatte Muskelzellen
   Beispiele für Aktivatorsequenzen für Gene in glatten Muskelzellen sind bereits unter 4.1. aufgeführt.
b) aktivierte Endothelzellen
   Beispiele für Proteine, welche besonders in aktivierten Endothelzellen gebildet werden, sind von Burrows et al. (Pharmac. Therp. 64, 155 (1994)) und Plate et al. (Brain Pathol. 4, 207 (1994)) beschrieben worden. Im besonderen zählen zu diesen in Endothelzellen verstärkt auftretenden Proteinen beispielsweise:
   - Hirn-spezifischer, endothelialer Glucose-1-Transporter
      Endothelzellen des Hirns zeichnen sich durch eine sehr starke Expression dieses Transporters aus, um den transendothelialen Transport von D-Glucose in das Hirn zu bewerkstelligen (Gerhart et al., J. Neurosci. Res. 22, 464 (1989)). Die Promotorsequenz wurde von Murakami et al. (J. Biol. Chem. 267, 9300 (1992)) beschrieben.
   - Endoglin
      Endoglin scheint ein nicht signalübertragender Rezeptor des TGFβ zu sein (Gougos et al., J. Biol. Chem. 265, 8361 (1990), Cheifetz, J. Biol. Chem. 267, 19027 (1992), Moren et al., BBRC 189, 356 (1992)). In geringen Mengen kommt er auf normalem Endothel vor, ist jedoch verstärkt exprimiert auf proliferierendem Endothel (Westphal et al., J. Invest. Derm. 100, 27 (1993), Burrows et al., Pharmac. Ther. 64, 155 (1994)). Die Promotorsequenz wurde von Bellon et al. (Eur. J. Immunol. 23, 2340 (1993)) und Ge et al. (Gene 138, 201 (1994)) beschrieben.
   - VEGF-Rezeptoren
      Zwei Rezeptoren werden unterschieden (Plate et al., Int. J. Cancer 59, 520 (1994)):
      * VEGF-Rezeptor-1 (flt-1)
         (de Vries et al., Science 255, 989 (1992))
         (enthält im zytoplasmischen Teil eine fms-ähnliche Tyrosinkinase) und der
      * VEGF-Rezeptor-2 (flk-1, KDR)
         (Terman et al., BBRC 187, 1579 (1992))
         (enthält im zytoplasmischen Teil eine Tyrosinkinase).
      Beide Rezeptoren sind fast ausschließlich auf endothelialen Zellen (Senger et al., Cancer Metast. Rev. 12, 303 (1993)) anzutreffen.
   - andere endothelspezifische Rezeptortyrosinkinasen
      * til-1 oder til-2
         (Partanen et al., Mol. Cell Biol. 12, 1698 (1992), Schnürch and Risau, Development 119, 957 (1993), Dumont et al., Oncogene 7, 1471 (1992))
      * B61-Rezeptor (Eck-Rezeptor)
         (Bartley et al., Nature 368, 558 (1994), Pandey et al., Science 268, 567 (1995), van der Geer et al., Ann. Rev. Cell Biol. 10, 251 (1994))
   - B61
      Das B61-Molekül stellt den Liganden dar für den B61-Rezeptor.
      (Holzman et al., J. Am. Soc. Nephrol. 4, 466 (1993), Bartley et al., Nature 368, 558 (1994))
   - Endothelin, im speziellen
      * Endothelin B
         (Oreilly et al., J. Cardiovasc. Pharm. 22, 18 (1993), Benatti et al., J. Clin. Invest. 91, 1149 (1993), O'Reilly et al., BBRC 193, 834 (1993). Die Promotorsequenz wurde von Benatti et al., J. Clin. Invest. 91, 1149 (1993) beschrieben.
      * Endothelin-1
         (Yanasigawa et al., Nature 332, 411 (1988).
         Die Promotorsequenz wurde von Wilson et al., Mol. Cell. Biol. 10, 4654 (1990) beschrieben.
   - Endothelin-Rezeptoren, insbesondere der Endothelin-B-Rezeptor
      (Webb et al., Mol. Pharmacol. 47, 730 (1995), Haendler et al., J. Cardiovasc. Pharm. 20, 1 (1992)).
   - Mannose-6-Phosphat-Rezeptoren
      (Perales et al., Eur. J. Biochem. 226, 225 (1994).
      Die Promotorsequenzen sind von Ludwig et al. (Gene 142, 311 (1994)), Oshima et al. (J. Biol. Chem. 263, 2553 (1988)) und Pohlmann et al. (PNAS USA 84, 5575 (1987)) beschrieben worden.
   - von Willebrand Faktor
      Die Promotorsequenz wurde von Jahroudi und Lynch (Mol. Cell. Biol. 14, 999 (1994), Ferreira et al., Biochem. J. 293, 641 (1993) und Aird et al., PNAS USA 92, 4567 (1995)) beschrieben.
   - IL-1α, IL-1β.
      IL-1 wird von aktivierten Endothelzellen produziert (Warner et al., J. Immunol. 139, 1911 (1987)).
      Die Promotorsequenzen wurden von Hangen et al., Mol. Carcinog. 2, 68 (1986), Turner et al., J. Immunol. 143, 3556 (1989), Fenton et al., J. Immunol. 138, 3972 (1987), Bensi et al., Cell Growth Diff. 1, 491 (1990), Hiscott et al., Mol. Cell. Biol. 13, 6231 (1993) und Mori et al., Blood 84, 1688 (1994) beschrieben
   - IL-1-Rezeptor
      Die Promotorsequenz wurde von Ye et al., PNAS USA 90, 2295 (1993) beschrieben.
   - Vascular Cell Adhesion Molecule (VCAM-1)
      Die Expression von VCAM-1 in Endothelzellen wird aktiviert durch Lipopolysaccharide, TNF-α (Neish et al., Mol. Cell. Biol. 15, 2558 (1995)), IL-4 (lademarko et al., J. Clin. Invest. 95, 264 (1995)) und IL-1 (Marni et al., J. Clin. Invest. 92, 1866 (1993)).
      Die Promotorsequenz von VCAM-1 wurde beschrieben von Neish et al., Mol. Cell. Biol. 15, 2558 (1995), Ahmad et al., J. Biol. Chem. 270, 8976 (1995), Neish et al., J. Exp. Med. 176, 1583 (1992), lademarco et al., J. Biol. Chem. 267, 16323 (1992) und Cybulsky et al., PNAS USA 88, 7859 (1991).
   - synthetische Aktivatorsequenz
      Als Alternative zu natürlichen endothelspezifischen Promotoren lassen sich auch synthetische Aktivatorsequenzen verwenden, die aus oligomerisierten Bindestellen für Transkriptionsfaktoren, die preferentiell oder selektiv in Endothelzellen aktiv sind, bestehen.
      Ein Beispiel hierfür ist der Transkriptionsfaktor GATA-2, dessen Bindestelle im Endothelin-1 Gen 5'-TTATCT-3' ist (Lee et al., Biol. Chem. 266, 16188 (1991), Dorfmann et al., J. Biol. Chem. 267, 1279 (1992) und Wilson et al., Mol. Cell. Biol. 10, 4854 (1990)).
c) aktivierte Makrophagen und/oder aktivierte Lymphozyten
   Als Aktivatorsequenz im Sinne dieser Erfindung sind außerdem Promotorsequenzen der Gene für Proteine zu verstehen, welche bei der Immunreaktion in Makrophagen und/oder in Lymphozyten verstärkt gebildet werden. Hierzu gehören beispielsweise:
   * IL-1 (Bensi et al., Gene 52, 95 (1987), Fibbe et al., Blut 59, 147 (1989))
   * IL-1-Rezeptor (Colotta et al., Immunol. Today 15, 562 (1994), Sims et al., Clin. Immunol. Immunopath. 72, 9 1994), Ye et al., PNAS USA 90, 2295 (1993))
   * IL-2 (Jansen et al. CII 39, 207 (1994), Ohbo et al., J. Biol. Chem. 270, 7479 (1995))
   * IL-2-Rezeptor (Semenzato et al., Int. J. Clin Lab. Res. 22, 133 (1992))
   * IFN γ (Kirchner, DMW 111, 64 (1986), Lehmann et al., J. Immunol. 153, 165 (1994))
   * IL-4 (Paul, Blood 77, 1859 (1991), te Velde et al., Blood 76, 1392 (1990))
   * IL-4-Rezeptor (Vallenga et al., Leukemia 7, 1131 (1993), Galizzi et al., Int. Immunol. 2, 669 (1990))
   * IL-3 (Frendl, Int. J. Immunopharm. 14, 421 (1992))
   * IL-5 (Azuma et al., Nucl. Acid Res. 14, 9149 (1986), Yokota et al., PNAS 84, 7388 (1987))
   * IL-6 (Brack et al., Int. J. Clin. Lab. Res. 22, 143 (1992)) or
   * LIF (Metcalf, Int. J. Cell Clon. 9, 95 (1991), Samal, BBA 1260, 27 (1995))
   * IL-7 (Joshi et all, 21, 681 (1991))
   * IL-10 (Benjamin et al., Leuk. Lymph. 12, 205 (1994), Fluchiger et al., J. Exp. Med. 179, 91 (1994))
   * IL-11 (Yang et al., Biofactors 4, 15 (1992))
   * IL-12 (Kiniwa et al., J. Clin. Invest. 90, 262 (1992), Gatelay, Cancer Invest. 11, 500 (1993))
   * IL-13 (Punnonen et al., PNAS 90, 3730 (1993), Muzio et al., Blood 83, 1738 (1994))
   * GM-CSF (Metcalf, Cancer 15, 2185 (1990))
   * GM-CSF Rezeptor (Nakagawa et al., J. Biol. Chem. 269, 10905 (1994))
   * Adhäsionsproteine wie Integrin beta2 protein (Nueda et al., J. Biol. Chem. 268, 19305 (1993))

   Promotorsequenzen für diese Proteine sind durch folgende Veröffentlichungen zugänglich:
   * IL-1-Rezeptor
      (Ye et al., PNAS USA 90, 2295 (1993))
   * IL-1α
      (Hangen et al., Mol. Carcinog. 2, 68 (1986), Turner et al., J. Immunol. 143, 3556 (1989), Mori et al., Blood 84, 1688 (1994))
   * IL-1β
      (Fenton et al., J. Immunol. 138, 3972 (1987), Bensi et al., Cell Growth Diff. 1, 491 (1990), Turner et al., J. Immunol. 143, 3556 (1989), Hiscott et al., Mol. Cell. Biol. 13, 6231 (1993))
   * IL-2
      (Fujita et al., Cell 46, 401 (1986), Hama et al., J. Exp. Med. 181, 1217 (1995), Kant et al., Lymph. Rec. Interact. 179 (1989), Kamps et al., Mol. Cell. Biol. 10, 5464 (1990), Williams et al., J. Immunol. 141, 662 (1988), Brunvand, FASEB J. 6, A998 (1992))
   * IL-2-Rezeptor
      (Ohbo et al., J. Biol. Chem. 270, 7479 (1995), Shibuya et al., Nucl. Acids Res. 18, 3697 (1990), Lin et al., Mol. Cell. Biol. 13, 6201 (1993), Williams et al., J. Immunol. 141, 662 (1988))
   * IFNγ
      (Ye et al., J. Biol. Chem. 269, 25728 (1994))
   * IL-4
      (Rooney et al., EMBO J. 13, 625 (1994), Hama et al., J. Exp. Med. 181, 1217 (1995), Li-Weber et al., J. Immunol. 153, 4122 (1994), 148, 1913 (1992), Min et al., J. Immunol. 148, 1913 (1992), Abe et al., PNAS 89, 2864 (1992))
   * IL-4-Rezeptor
      (Beckmann et al., Chem. Immunol. 51, 107 (1992), Ohara et al., PNAS 85, 8221 (1988))
   * IL-3
      (Mathey-Prevot et al., PNAS USA 87, 5046 (1990), Cameron et al., Blood 83, 2851 (1994), Arai et al., Lymphokine Res. 9, 551 (1990))
   * IL-3-Rezeptor (a-subunit)
      (Miyajima et al., Blood 85, 1246 (1995), Rapaport et al., Gene 137, 333 (1993), Kosugi et al., BBRC 208, 360 (1995))
   * IL-3-Rezeptor (β-subunit)
      (Gorman et al., J. Biol. Chem. 267, 15842 (1992), Kitamura et al., Cell 66, 1165 (1991), Hayashida et al., PNAS USA 87, 9655 (1990))
   * IL-5
      (Lee et al., J. Allerg. Clin. Immunol. 94, 594 (1994), Kauhansky et al., J. Immunol. 152, 1812 (1994), Staynov et al., PNAS USA 92, 3606 (1995))
   * IL-6
      (Lu et al., J. Biol. Chem. 270, 9748 (1995), Gruss et al., Blood 80, 2563 (1992), Ray et al., PNAS 85, 6701 (1988), Droogmans et al., DNA-Sequence 3, 115 (1992), Mori et al., Blood 84, 2904 (1994), Liberman et al., Mol Cell. Biol. 10, 2327 (1990), Ishiki et al., Mol. Cell. Biol. 10, 2757 (1990))
   * IL-7
      (Pleiman et al., Mol. Cell. Biol. 11, 3052 (1991), Lapton et al., J. Immunol. 144, 3592 (1990))
   * IL-8
      (Chang et al., J. Biol. Chem. 269, 25277 (1994), Sprenger et al., J. Immunol. 153, 2524 (1994))
   * IL-10
      (Kim et al., J. Immunol. 148, 3618 (1992), Platzer et al., DNA Sequence 4, 399 (1994), Kube et al., Cytokine 7, 1 (1995))
   * IL-11
      (Yang et al., J. Biol. Chem. 269, 32732 (1994))
   * GM-CSF
      (Nimer et al., Mol. Cell. Biol. 10, 6084 (1990), Staynov et al., PNAS USA 92, 3606 (1995), Koyano-Nakayawa et al., Int. Immunol. 5, 345 (1993), Ye et al., Nucl. Acids Res. 22, 5672 (1994))
   * GM-CSF-Rezeptor (α-Kette)
      (Nakagawa et al., J. Biol. Chem. 269, 10905 (1994))
   * Makrophagen-Colony Stimulating Factor (M-CSF)-Rezeptor
      (Yue et al., Mol. Cell. Biol. 13, 3191 (1993), Zhang et al., Mol. Cell. Biol. 14, 373 (1994))
   * Typ I und II Makrophagen Scavenger Rezeptoren
      (Moulton et al., Mol. Cell. Biol. 14, 4408 (1994))
   * IL-13
      (Staynov et al., PNAS USA 92, 3606 (1995))
   * LIF
      (Gough et al., Ciba Found. Symp. 167, 24 (1992), Stahl et al., Cytokine 5, 386 (1993))
   * Interferon Regulatory Factor 1, dessen Promotor durch IL-6 wie auch durch IFNγ oder beta stimuliert wird
      (Harrock et al., EMBO J. 13, 1942 (1994))
   * IFNγ Responsive Promotor
      (Lamb et al., Blood 83, 2063 (1994))
   * IFNγ
      (Hardy et al., PNAS 82, 8173 (1985))
   * MAC-1
      (Dziennis et al., Blood 85, 319 (1995), Bauer et al., Hum. Gene Ther. 5, 709 (1994), Hickstein et al., PNAS USA 89, 2105 (1992))
   * LFA-1α
      (Nueda et al., J. Biol. Chem. 268, 19305 (1993), Agura et al., Blood 79, 602 (1992), Cornwell et al., PNAS USA 90, 4221 (1993))
   * p150,95
      (Noti et al., DNA and Cell Biol. 11, 123 (1992), Lopezcabrera et al., J. Biol. Chem. 268, 1187 (1993))

### 5.2. Auswahl der Wirksubstanz zur Inhibition der Gerinnung

Als Wirksubstanz im Sinne dieser Erfindung ist eine DNA-Sequenz zu verwenden, welche ein Protein kodiert, welches direkt oder indirekt die Thrombozytenaggregation oder einen Blutgerinnungsfaktor inhibiert oder die Fibrinolyse stimuliert.

Eine derartige Wirksubstanz wird als Gerinnungshemmer bezeichnet. Als Gerinnungshemmer sind Gene für beispielsweise Plasminogenaktivatoren (PA), so der Gewebe-PA (tPA) oder der Urokinase-ähnliche PA (uPA) oder Protein C, Antithrombin-III, C-1S-Inhibitor, α1-Antitrypsin, der Tissue Factor Pathway Inhibitor (TFPI) oder Hirudin einzusetzen. Die DNA-Sequenzen für diese Gerinnungshemmer wurden bereits im Abschnitt 4.3, beschrieben:

### 5.3. Kombination zweier gleicher oder unterschiedlicher Wirksubstanzen zur Inhibition der Gerinnung

Gegenstand der Erfindung ist des weiteren ein Wirkstoff, in welchem eine Kombination der DNA-Sequenzen von zwei gleichen gerinnungshemmenden Substanzen (A,A) oder zwei unterschiedlichen gerinnungshemmenden Substanzen (A,B) vorliegt. Zur Expression beider DNA-Sequenzen ist vorzugsweise die cDNA einer "internal ribosomal entry site" (IRES) als regulatorisches Element zwischengeschaltet.

Aktivator-
sequenz
(UAS)

zellzyklus-
reguliertes
Promotor-
modul

Wirksubstanz
(Gerinnungs-
hemmer)
A

internal
ribosome
entry
site

Wirksubstanz
(Gerinnungs-
hemmer)
A oder B

Derartige IRES wurden beispielsweise von Montford und Smith (TIG 11, 179 (1995), Kaufman et al., Nucl. Acids Res. 19, 4485 (1991), Morgan et al., Nucl. Acids Res. 20, 1293 (1992, Dirks et al., Gene 128, 247 (1993), Pelletier und Sonenberg, Nature 334, 320 (1988) und Sugitomo et al., BioTechn. 12, 694 (1994) beschrieben.

So kann die cDNA der IRES-Sequenz des Poliovirus (Position ≤ 140 bis ≥ 630 des 5' UTR (Pelletier und Sonenberg, Nature 334, 320 (1988)) zur Verknüpfung der DNA der antithrombotischen Substanz A (am 3' Ende) und der DNA der antithrombotischen Substanz B (am 5' Terminus) verwendet werden.

Eine derartige Kombination zweier gleicher oder unterschiedlicher Gene bewirkt eine additive Wirkung (bei gleichen Genen) oder eine synergistische Wirkung der gewählten antithrombotischen Substanzen.

### 5.4. Auswahl des Liganden zur Inhibition der Gerinnung

Als Ligand für virale oder nicht-virale Vektoren, beispielsweise in kolloidalen Dispersionen, enthaltend Polylysin-Ligand-Konjugaten, werden Substanzen bevorzugt, welche an die Zelloberfläche von glatten Muskelzellen oder von proliferierenden Endothelzellen oder von aktivierten Makrophagen und/oder Lymphozyten binden.
a) Liganden für glatte Muskelzellen
   Beispiele für Liganden, die an glatte Muskelzellen binden, wurden schon im Abschnitt 4.4. aufgeführt.
b) Liganden für aktivierte Endothelzellen
   Hierzu gehören im Sinne der Erfindung Antikörper oder Antikörperfragmente, gerichtet gegen Membranstrukturen von Endothelzellen wie sie beispielsweise von Burrows et al. (Pharmac. Ther. 64, 155 (1994)), Hughes et al. (Cancer Res. 49, 6214 (1989) und Maruyama et al. (PNAS-USA 87, 5744 (1990) beschrieben wurden. Insbesondere zählen hierzu Antikörper gegen die VEGF-Rezeptoren.
   Die murinen monoklonalen Antikörper sind bevorzugt in humanisierter Form einzusetzen. Die Humanisierung erfolgt in der im Abschnitt 4.4. dargestellten Weise. Antikörperfragmente werden entsprechend dem Stand der Technik hergestellt, beispielsweise in der in Abschnitt 4.4. beschriebenen Weise.
   Zu den Liganden gehören des weiteren alle Wirkstoffe, welche an Membranstrukturen oder Membranrezeptoren auf Endothelzellen binden. Beispielsweise gehören hierzu Substanzen, die endständig Mannose enthalten des weiteren IL-1 oder Wachstumsfaktoren oder deren Fragmente bzw. Teilsequenzen von ihnen, die an Rezeptoren exprimiert durch Endothelzellen binden, wie beispielweise PDGF, bFGF, VEGF, TGFβ (Pusztain et al., J. Pathol. 169, 191 (1993)).
   Des weiteren gehören hierzu Adhäsionsmoleküle, welche an aktivierte und/oder proliferierende Endothelzellen binden. Derartige Adhäsionsmoleküle, wie beispielsweise SLex, LFA-1, MAC-1, LECAM-1 oder VLA-4, wurden bereits beschrieben (Übersichten bei Augustin-Voss et al., J. Cell Biol. 119, 483 (1992), Pauli et al., Cancer Metast. Rev. 9, 175 (1990), Honn et al., Cancer Metast. Rev. 11, 353 (1992)).
c) Liganden für aktivierte Makrophagen und/oder aktivierte Lymphozyten
   Zu den Liganden im Sinne der Erfindung gehören des weiteren Substanzen, welche an die Oberfläche von Immunzellen spezifisch binden. Hierzu gehören Antikörper oder Antikörperfragmente gerichtet gegen Membranstrukturen von Immunzellen, wie sie beispielsweise von Powelson et al., Biotech. Adv. 11, 725 (1993) beschrieben wurden.
   Desweiteren gehören zu den Liganden auch monoklonale oder polyklonale Antikörper oder Antikörperfragmente, die mit ihren konstanten Domänen an Fcγ- oder µ-Rezeptoren von Immunzellen binden (Rojanasakul et al., Pharm. Res. 11, 1731 (1994)).
   Auch hier werden die murinen monoklonalen Antikörper bevorzugt in humanisierter Form eingesetzt (siehe Abschnitt 4.4.) und Fragmente, beispielsweise mit der in Abschnitt 4.4. zitierten Methodik, hergestellt.
   Zu den Liganden gehören des weiteren alle Substanzen, welche an Membranrezeptoren auf der Oberfläche von Immunzellen binden. Beispielsweise gehören hierzu Wachstumsfaktoren, wie Zytokine, EGF, TGF, FGF oder PDGF, oder deren Fragmente bzw. Teilsequenzen von ihnen, die an Rezeptoren exprimiert durch derartige Zellen binden.

### 5.5. Herstellung des Wirkstoffes zur Inhibition der Gerinnung

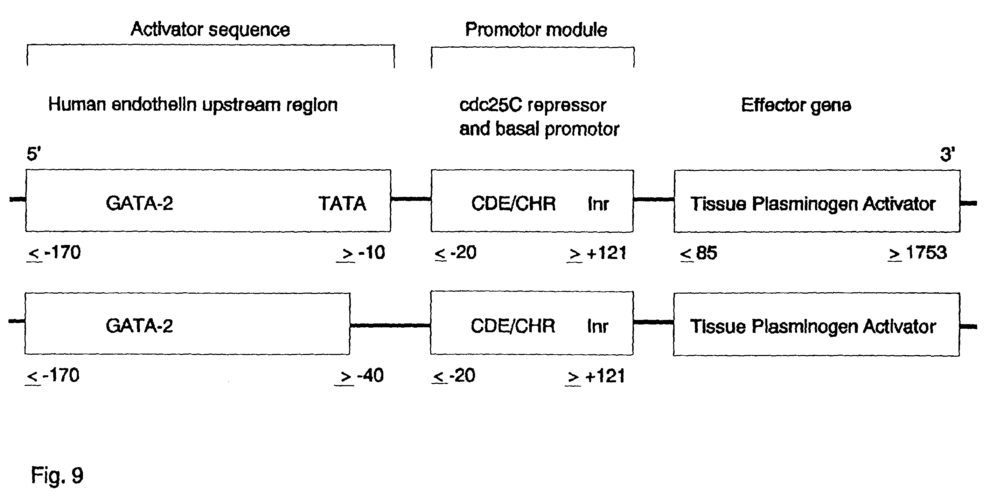

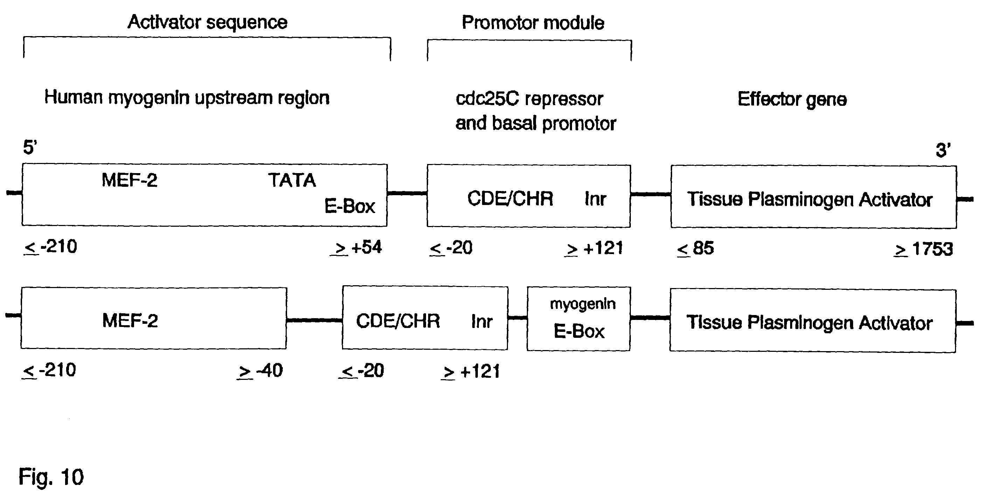

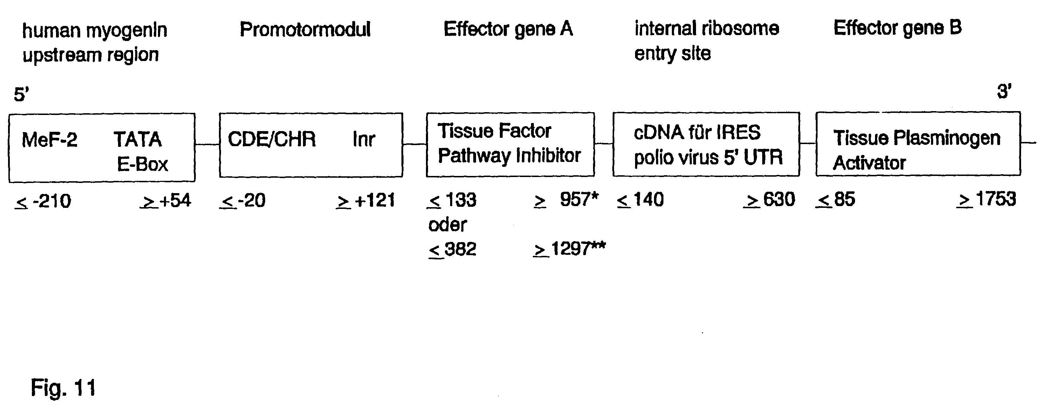

Die Herstellung des erfindungsgemäßen Wirkstoffes wird anhand folgender Beispiele näher beschrieben:
a) Konstruktion des chimären Promotors Endothelin 1CDE-CHR-Inr
   Der humane Endothelin-1 Promotor (Position ≤ -170 bis ≥ -10, Wilson et al., Mol. Cell. Biol. 10, 4854 (1990)) oder eine um die TATA-Box verkürzte Variante (Position ≤ -170 bis ≥ 40) werden an ihrem 3' Ende mit dem 5'-Terminus des CDE-CHR-Inr Moduls (Position ≤ -20 bis ≥ +121) des humanen cdc25C-Gens (Lucibello et al., EMBO J., 14, 132 (1995)) verknüpft (Fig. 9). Die Verknüpfung erfolgt mit Hilfe von dem Fachmann bekannten und käuflichen Enzymen.
b) Konstruktion eines Plasmids enthaltend den chimären Promotor Endothelin-1-CDE-CHR-Inr im zentralen Bestandteil des Wirkstoffes
   Die beschriebene chimäre Endothelin-1 Promotormodul-Transkriptionseinheit wird an ihren 3' Enden mit dem 5'-Terminus einer DNA, die den kompletten kodierenden Bereich des Tissue Plasminogen Activators (Position ≤ 85 bis ≥ 1753, Pennica et al., Nature 301, 214 (1983)) enthält, verknüpft (Fig. 9). Diese DNA enthält auch die für eine Sekretion notwendige Signalsequenz. Transkriptionskontrolleinheiten und die DNA für Tissue Plasminogen Activator werden in pUC19/19 oder Bluescript-abgeleiteten Plasmidvektoren einkloniert, die direkt oder in kolloidalen Dispersionssystemen für eine in vivo Applikation genutzt werden können. Alternativ können die chimären Gene in virale Vektoren oder andere geeignete Vektoren transferiert und injiziert werden.
c) Konstruktion des chimären Promotors Myogenin-CDE-CHR-Inr
   Der humane Myogenin-Promotor (Pos. ≤ -210 bis ≥ +54, der von Salmin et al., J. Cell Biol. 115, 905 (1991 publizierten DNA-Sequenz) wird an seinem 3'-Ende mit dem 5'-Terminus des CDE-CHR-Inr Moduls des humanen cdc25C-Gens (Pos. ≤ -20 bis ≥ +121, der von Lucibello et al., EMBO J. 14, 132 (1995) publizierten Sequenz) verknüpft (siehe Fig. 10). Die Verknüpfung erfolgt mit Hilfe von dem Fachmann bekannten und käuflichen Enzymen. Außerdem werden verschiedene Fragmente der Myogenin-Promotorsequenz verwendet (siehe Fig. 10). So wird die TATA-Box enthaltende DNA-Sequenz des Myogenin-Promotors verwendet. Jedoch kann gleichermaßen auch die Promotorsequenz Position ≤ -210 bis ≥ -40 zur Anwendung kommen.
d) Konstruktion eines Plasmids enthaltend den chimären Promotor Myogenin-CDE-CHR-Inr im zentralen Bestandteil des Wirkstoffes
   Die so hergestellte chimäre Myogenin-Promotormodul-Transkriptionskontrolleinheit wird an ihrem 3' Ende mit dem 5'-Terminus einer DNA, die den kompletten kodierenden Bereich des Tissue Plasminogen Activators enthält, verknüpft (siehe Fig. 10). Diese DNA enthält auch die für eine Sekretion notwendige Signalsequenz. Transkriptionskontrolleinheiten und die DNA für den Tissue Plasminogen Activator werden in pUC18/19 oder Bluescript-abgeleiteten Plasmidvektoren einkloniert, die direkt oder in kolloidalen Dispersionssystemen für eine in vivo Applikation genutzt werden können. Alternativ können die chimären Gene in virale Vektoren oder andere geeignete Vektoren transferiert und injiziert werden.
e) Konstruktion eines Plasmids enthaltend zwei Gene für Wirksubstanzen
   Die, wie unter c) beschriebene, Myogenin-CDE-CHR-Inr-Transkriptionseinheit wird an ihrem 3' Ende mit dem 5' Ende der DNA für den Tissue Factor Pathway Inhibitor (TFPI, Position ≤ 133 bis ≥ 957; Wun et al., J. Biol. Chem. 263, 6001 (1988) oder Position ≤ 382 bis ≥ 1297; Girard et al., Thromb. Res. 55, 37 (1989)) verknüpft. Die Verknüpfung erfolgt mit Hilfe von dem Fachmann bekannten und käuflichen Enzymen.
   Das 3' Ende der DNA für TFPI wird nunmehr verknüpft mit dem 5' Ende der cDNA der internal ribosome entry site (Position ≤ 140 bis ≥ 630; Pelletier und Sonnenberg, Nature 334, 320 (1988)) und ausschließend deren 3' Ende mit dem 5' Ende der DNA für den Tissue Plasminogen Activator verknüpft (siehe Fig. 11). Dieser so hergestellte Wirkstoff wird ausschließlich in puc18/19 oder in Bluescript-abgeleiteten Plasmidvektoren einkloniert, die direkt oder in kolloidalen Dispersionssystemen für eine in vivo Applikation genutzt werden können. Alternativ können die chimären Gene in virale Vektoren oder andere geeignete Vektoren transferiert und injiziert werden.

### 6. Wirkung des Wirkstoffes auf glatte Muskelzellen und/oder auf die Gerinnung

Ein Wirkstoff gemäß der vorliegenden Erfindung ermöglicht nach lokaler oder systemischer, bevorzugt intravenöser oder intraarterieller Gabe eine vorwiegende, wenn nicht ausschließliche Wirkung an solchen glatten Muskelzellen, welche durch Schäden oder Verletzungen des Gefäßes (insbesondere der Endothelschicht) und gegebenenfalls nach Wanderung in die Intima vom Gefäßvolumen her direkt zugänglich sind.

Durch die Kombination aus gewebespezifischer Aktivatorsequenz und zellzyklusreguliertem Repressormodul ist gewährleistet, daß der Zellzyklusinhibitor überwiegend oder ausschließlich in sich teilenden glatten Muskelzellen aktiviert wird.

Durch die erfindungsgemäße Verwendung von mutierten Zellzyklusinhibitoren ist deren längerfristige proliferationshemmende Wirkung gewährleistet.

Ein Wirkstoff gemäß der vorliegenden Erfindung ermöglicht des weiteren, daß nach lokaler (z.B. in Gewebe, Körperhöhlen oder Gewebezwischenräumen) oder nach systemischer, bevorzugt intravenöser oder intraarterieller Gabe, vorwiegend, wenn nicht ausschließlich, glatte Muskelzellen, aktivierte proliferierende Endothelzellen, aktivierte Lymphozyten oder aktivierte Makrophagen die antithrombotische Substanz exprimieren und diese somit am Ort der Entstehung der Thrombose freigesetzt wird.

Da der Wirkstoff sowohl durch seine Zell- als auch Zellzyklusspezifität ein hohes Maß an Sicherheit verspricht, kann er auch in hohen Dosierungen und, falls notwendig, mehrmals in Abständen von Tagen oder Wochen zur Prophylaxe oder zur Therapie von Gefäßverschlüssen, verursacht durch proliferierende glatte Muskelzellen und/oder zur Prophylaxe und/oder Therapie von Thrombosen angewandt werden.

Legende zu Fig. 1 - 11:
Fig. 1:
   Nukleotidsequenz des cdc25C - Promotorbereichs mit den *in vivo* gefundenen Proteinbindungsstellen (genomisches DMS Footprinting; (gefüllte Kreise): vollständiger konstitutiver Schutz; o (offene Kreise: partieller konstitutiver Schutz; * (Sternchen): zellzyklusregulierter, G1-spezifischer Schutz). CBS: constitutive binding site; CDE: cell cycle-dependent element. Grau unterlegte Bereiche zeigen die Y_{C}-Boxen (NF-Y Bindestellen) an. Startstellen sind durch gefüllte Quadrate markiert.
Fig. 2:
   Derepression des cdc25C-Promotors spezifisch in G₀ durch Mutation des cdc.
Fig. 3:
   Schematische Darstellung der cdc25C Enhancer-Regulation durch das CDE.
Fig. 4:
   G₀ / G₁ - spezifische Repression des SV40-Enhancers durch das CDE.
Fig. 5:
   Homologien im CDE-CHR-Bereich und den 5' gelegenen Yc-Boxen in den cdc25C, cyclin A und cdc2-Promotoren
Fig. 6:
   Chimäre Konstrukte bestehend aus verschiedenen Anteilen des humanen Myogenin (Myf-4) Promotors, dem 3' fusionierten Promotormodul mit den CDE und CHR Repressorelementen sowie einer DNA für humane β-glucuronidase (kompletter kodierender Bereich, Position ≤ 27 bis ≥ 1982) als Effektor (Oshima et al., PNAS USA 84, 685 (1987)). Positionsangaben beziehen sich auf die Angaben von Salminen et al., J. Cell Biol. 115, 905 (1991) für das Myogenin Gen bzw. auf das von Lucibello et al., EMBO J. 14, 132 (1995) verwendete System für cdc25C.
Fig. 7:
   Ersatz der homologen Signalsequenz der β-glucoronidase durch eine heterologe Signalsequenz (des humanen Immunglobulins). Positionsangabe der Signalsequenz (MGWSCIILFLVATAT) des Immunglobulins (HuVHcAMP) beziehen sich auf Riechmann et al., Nature 332, 323 (1988).
   Alternative: Einbau des Signalpeptids des Ig zur besseren extrazellulären Ausschleusung der β-glucuronidase, siehe (B).
Fig. 8:
   Chimäre Konstrukte bestehend aus verschiedenen Anteilen des humanen Myogenin (Myf-4) Promotors, dem 3' fusionierten Promotormodul mit den CDE und CHR Repressorelementen sowie einer DNA für humane β-glucuronidase, der internal ribosome entry site als regulierende Nukleotidsequenz und der DNA für Tissue Plasminogen Activator. Die Positionsangaben beziehen sich auf Salminen et al., J. Cell Biol. 115, 905 (1991) für das Myogenin, Lucibello et al. EMBO J. 14, 132 (1995) für das CDE/CHR-lnr Element, Oshima et al., PNAS USA 84, 685 (1987) für β-glucuronidase, Riechmann et al., Nature 332, 323 (1988) für die Signalsequenz des Immunoglobulins, Pelletier und Sonenberg, Nature 334, 320 (1988) für die Internal ribosome entry site des Poliovirus und auf Pennica et al., Nature 301, 214 (1983) für den humanen Tissue Plasminogen Activator.
Fig. 9:
   Chimäre Konstrukte bestehend aus verschiedenen Anteilen des humanen Endothelin-1 Promotors, dem 3' fusionierten Promotormodul mit den CDE und CHR Repressorelementen sowie einer DNA für den humanen Tissue Plasminogen Activator (kompletter kodierender Bereich, Pennica et al., Nature 301, 214 (1983)) als Effektor. Positionsangaben beziehen sich auf die Angaben von Wilson et al., Mol. Cell. Biol. 10, 4854 (1990) für das Endothelin-1 Gen bzw. auf das von Lucibello et al., EMBO J. 14, 132 (1995) verwendete System für cdc25C.
Fig. 10:
   Chimäre Konstrukte bestehend aus verschiedenen Anteilen des humanen Myogenin (Myf-4) Promotors, dem 3' fusionierten Promotormodul mit den CDE und CHR Repressorelementen sowie einer DNA für den humanen Tissue Plasminogen Activator (kompletter kodierender Bereich) als Effektor. Positionangaben beziehen sich auf die Angaben von Salminen et al., J. Cell Biol. 115, 905 (1991) für das Myogenin Gen bzw. auf das von Lucibello et al., EMBO J. 14, 132 (1995) verwendete System für cdc25C und auf die Angaben von Pennica et al., Nature 301, 214 (1983) für den Tissue Plasminogen Activator.
Fig. 11:
   Chimäre Konstrukte mit zwei Effektorgenen
   Positionsangabe für den Tissue Factor Pathway Inhibitor beziehen sich auf
   *) Wun et al., J. Biol. Chem. 263, 6001 (1988)
   **) Girard et al., Thromb. Res. 55, 37 (1989)
   und für die internal ribosome entry site (IRES) cDNA basierend auf Pelletier
   und Sonenberg, Nature 334, 320 (1988)

**Tabelle 1:**

| Rolle von CDE und CHR bei der zellzyklusregulierten Transkription von cdc25C, cyclin A und cdc2 | | | |
|---|---|---|---|
| ***Tab. 1*** | | | |
| | ***G***_{***0***} | ***Growing*** | ***Factor*** |
| ***wt*** | | | |
| cdc25C | 0.8 | 13.1 | 17.5 |
| | | | |
| cyclin A | 0.7 | 27.1 | 41.7 |
| cdc2 | 1.0 | 41.2 | 41.2 |
| ***mCDE(-13)*** | | | |
| cdc25C | 7.6 | 11.6 | 1.5 |
| | | | |
| cyclin A | 13.4 | 23.9 | 1.8 |
| cdc2 | 11.3 | 33.9 | 3.0 |
| ***mCHR(-6/-3)*** | | | |
| cdc25C | 14.4 | 21.0 | 1.5 |
| | | | |
| cyclin A | 15.5 | 28.3 | 1.8 |
| cdc2 | 18.6 | 38.6 | 2.1 |

Ergebnisse transienter Transfektionen in HIH3T3-Zellen sind als RLUs/1000 dargestellt. mCDE: mutiertes CDE (Pos. -13: G → T); mCHR: mutiertes CHR (Pos. -6 bis -3).

## Patentansprüche

1. Wirkstoff zur Prophylaxe oder Therapie von Erkrankungen des Blutgefäßsystems, dadurch charakterisiert, daß er ein DNA-Konstrukt enthält, welches aus einer Aktivatorsequenz, einem zellzyklusregulierten Promotormodul und einer DNA-Sequenz für eine Wirksubstanz besteht, welche einen Zellzyklusinhibitor und/oder einen Gerinnungshemmer darstellt.

2. Wirkstoff nach Anspruch 1., bei welchem das Promotormodul die Elemente CDE-CHR-Inr besitzt und die Positionen ≤ -20 bis ≥ +30 des cdc25C Promotorbereiches enthält (Nukleotidsequenz GGCTGGCGGAAGGTTT-GAATGGTCAACGCCTGCGG-CTGTTGATATTCTTG), wobei CDE das "cell cycle dependent element" (Nukleotidsequenz TGGCGG), CHR die "cell cycle gene homology region" (Nukleotidsequenz GTTTGAA) und Inr die Initiations-stelle (Position +1) sowie die für die Initiation wichtigen benachbarten Sequenzen darstellen.

3. Wirkstoff nach Anspruch 1. und 2. enthaltend eine Aktivatorsequenz (Promotor- oder Enhancersequenz), welche durch Transkriptionsfaktoren gebildet in glatten Muskelzellen, reguliert wird.

4. Wirkstoff nach Anspruch 3. enthaltend
- als Aktivatorsequenz den CMV-Enhancer, den CMV-Promotor oder den SV40 Promotor oder
- die Promotorsequenz für das Gen von Tropomyosin, α-Actin, α-Myosin, PDGF-Rezeptoren, FGF-Rezeptoren, MRF-4, Acetylcholinrezeptoren, Phosphofructinase A, Phosphoglyceratmutase, Troponin C, Myogenin, Endothelin-Rezeptoren oder Desmin oder
- als Aktivatorsequenz multiple Kopien einer Bindestelle für muskelspe-zifische HLH-Proteine (E-Box) oder für GATA-4 oder
- die Promotorsequenz für VEGF oder die Enhancersequenz für VEGF oder die DNA-Sequenz für c-Src oder für v-Src, welche das VEGF-Gen regulieren.

5. Wirkstoff nach Anspruch 1-4, bei welchem die DNA-Sequenz für die Wirksubstanz einen Zellzyklusinhibitor kodiert, der darstellt
- das Retinoblastomprotein pRb/p110, das p107 Protein oder das 130 Protein oder
- das p53 Protein oder
- das p21 Protein, das p16 Protein oder einen anderen "Cyclindependent Kinase (cdK)"- Inhibitor oder
- das GADD45 Protein oder
- das bak Protein oder
- ein zytostatisches oder zytotoxisches Protein oder
- ein Enzym für die Spaltung von Vorstufen von Zytostatika in Zytostatika.

6. Wirkstoff nach Anspruch 5.,
- bei welchem das Retinoblastomprotein (pRb/p110) durch Austausch der Aminosäuren in den Positionen 246, 350, 601, 605, 780, 786, 787, 800 und 804 nicht mehr phosphorylierbar wird, ohne jedoch seine Bindungsaktivität mit dem großen T-Antigen einzubüßen, beispielsweise, daß die Aminosäuren Thr-246, Ser-601, Ser-605, Ser-780, Ser-786, Ser-787 und Ser-800 mit Ala, der Aminosäure Thr-350 mit Arg und der Aminosäure Ser-804 mit Glu ausgetauscht werden oder
- bei welchem das p107 Protein in analoger Weise wie das pRb/110 mutiert ist oder
- bei welchem das p130 Protein in analoger Weise wie das pRb/110 mutiert ist.

7. Wirkstoff nach Anspruch 5., bei welchem das Protein p53 C-terminal verkürzt ist um das Serin 392.

8. Wirkstoff nach Anspruch 1-4, bei welchem der Zellzyklusinhibitor Perforin, Granzym, TNFα oder TNFβ ist.

9. Wirkstoff nach Anspruch 1-4., bei welchem der Zellzyklusinhibitor ein Enzym darstellt und dieses Enzym eine Herpes Simplex Virus Thymidin-Kinase, Cytosindeaminase, Varizella Zoster Virus Thymidin-Kinase, Nitroreduktase, β-Glucuronidase (im besonderen eine humane, pflanzliche oder bakterielle β-Glucuronidase), Carboxypeptidase (vorzugsweise von Pseudomonas), Lactamase (vorzugsweise von Bazillus cereus), Pyroglutamat-Aminopepti-dase, D-Aminopeptidase, Oxidase, Peroxidase, Phosphatase, Hydroxynitrillyase, Protease, Esterase oder eine Glycosidase ist, wobei die Signalsequenz für dieses Enzym homolog oder zur besseren zellulären Ausschleusung auch heterolog sein kann.

10. Wirkstoff nach Anspruch 9., bei welchem durch Punktmutationen der cDNA-Sequenz des Enzyms die lysosomale Speicherung verringert und die extrazelluläre Ausschleusung erhöht ist.

11. Wirkstoff nach Anspruch 1-10., welcher die DNA-Sequenzen von mehreren gleichen oder unterschiedlichen Zellzyklusinhibitoren enthält, wobei jeweils zwei DNA-Sequenzen durch eine DNA-Sequenz für die internal ribosome entry site miteinander verbunden sind.

12. Wirkstoff nach Anspruch 1-4., bei welchem die DNA-Sequenz für die Wirksubstanz einen Gerinnungshemmer kodiert.

13. Wirkstoff nach Anspruch 1- 10., bei welchem die DNA-Sequenz für einen Zellzyklusinhibitor ergänzt ist um die DNA-Sequenz für einen Gerinnungshemmer und diese beiden DNA-Sequenzen durch eine DNA-Sequenz für die internal ribosome entry site miteinander verbunden sind.

14. Wirkstoff nach Anspruch 12. und 13., bei welchem die DNA-Sequenz für den Gerinnungshemmer tPA, uPA, Hybridmoleküle von tPA und uPA, Protein C, Antithrombin III, TFPI, C-1 Inhibitor, α1-Antitrypsin oder Hirudin kodiert.

15. Wirkstoff nach Anspruch 1. und 2. enthaltend eine Aktivatorsequenz (Promotor- oder Enhancersequenz), welche durch Transkriptionsfaktoren gebildet in proliferierenden Endothelzellen reguliert wird.

16. Wirkstoff nach Anspruch 15, enthaltend
- die Promotorsequenz für den endothelialen Glucose-1-Transporter, für Endoglin, VEGF-Rezeptor-1 oder -2, Rezeptortyrosinkinase til-1 oder til-2, B61 Rezeptor, B61 Ligand, Endothelin, insbesondere Endothelin-B, -1, Mannose-6-Phosphat-Rezeptor, II-1α oder II-1β, 11-1-Rezeptor, VCAM-1, von Willebrand Faktor oder
- eine synthetische Aktivatorsequenz aus oligomerisierten Bindestellen für Transkriptionsfaktoren, die preferentiell oder selektiv in Endothelzellen aktiv sind wie GATA-2, dessen Bindungsstelle 5'-TTATCT-3' ist.

17. Wirkstoff nach Anspruch 1. und 2., enthaltend eine Aktivatorsequenz (Promotor- oder Enhancersequenz), welche durch Transkriptionsfaktoren reguliert wird, die im besonderen Maße gebildet werden bei der Aktivierung von und in Makrophagen oder in Lymphozyten.

18. Wirkstoff nach Anspruch 17., enthaltend eine Promotorsequenz für Cytokine oder deren Rezeptoren, im besonderen für Interleukin (II)-1 α, oder II-1β, 11-1-Rezeptor, II-2, II-2-Rezeptor, Interferon γ, II-3, II-3-Rezeptor, II-4, II-4-Rezeptor, II-5, II-6, II-7, II-8, II-9, II-10, II-11, II-12, II-13, M-CSF-Rezeptor, Makrophagen Scavenger Typ I oder Typ II Rezeptor, Interferon Regulatory Factor 1, IFN-γ Responsive Promotor, IFN-γ, Granulozyten-Makrophagen-koloniestimulierender Faktor (GM-CSF), GM-CSF-Rezeptor, Granulozytenkoloniestimulierender Faktor (G-CSF), Adhäsionsproteine, wie LFA-1, Mac-1 oder p150/95, oder Leukämie-inhibierender Faktor (LIF).

19. Wirkstoff nach Anspruch 15. -18., bei welchem die DNA-Sequenz für die Wirksubstanz einen Gerinnungshemmer kodiert.

20. Wirkstoff nach Anspruch 19., bei welchem die DNA-Sequenz für den Gerinnungshemmer den Tissue Plasminogen Activator (tPA), die Urokinase (uPA), Hybride von tPA und uPA, Protein C, Antithrombin III, den Tissue Factor Pathway Inhibitor, C-1 Inhibitor, α1-Antitrypsin oder Hirudin kodiert.

21. Wirkstoff nach Anspruch 1. - 4. und 15. - 20., welcher die DNA-Sequenzen von mehreren gleichen oder unterschiedlichen Gerinnungshemmern enthält, wobei jeweils zwei DNA-Sequenzen durch eine DNA-Sequenz für die internal ribosome entry site miteinander verbunden sind.

22. Wirkstoff nach Anspruch 1. - 21., eingefügt in einen Vektor.

23. Wirkstoff nach Anspruch 22., bei welchem der Vektor ein Virus ist.

24. Wirkstoff nach Anspruch 22., bei welchem das Virus ein Retrovirus, Adenovirus, Adeno-assoziiertes Virus, Herpes simplex Virus oder Vaccinia Virus darstellt.

25. Wirkstoff nach Anspruch 1. - 24. eingefügt in ein Plasmid.

26. Wirkstoff nach Anspruch 22. - 25., zubereitet in einem kolloidalen Dispersionssystem.

27. Wirkstoff nach Anspruch 26., bei welchem das kolloidale Dispersions system Liposome sind.

28. Wirkstoff nach Anspruch 26., bei welchem das kolloidale Dispersionssystem Polylysin-Liganden sind.

29. Wirkstoff nach Anspruch 22. - 28. ergänzt um einen Liganden, welcher an Membranstrukturen von glatten Muskelzellen, aktivierten Endothelzellen, aktivierten Makrophagen oder aktivierten Lymphozyten bindet.

30. Wirkstoff nach Anspruch 22), bei welchem der Ligand
- ein polyklonaler oder monoklonaler Antikörper oder ein Antikörperfragment hiervon ist, der mit seinen variablen Domänen spezifisch an die Membranstruktur von glatten Muskelzellen, aktivierten Endothelzellen, aktivierten Makrophagen oder aktivierten Lymphozyten bindet oder
- ein Zytokin oder Wachstumsfaktor oder ein Fragment bzw. eine Teilsequenz hiervon ist, der an Rezeptoren auf glatten Muskelzellen, aktivierten Endothelzellen, aktivierten Makrophagen oder aktivierten Lymphozyten bindet oder
- ein Adhäsionsmolekül darstellt, wie SLeX, LFA-1, MAC-1, LECAM-1 oder VLA-4.

31. Wirkstoff nach Anspruch 30), bei welchem die Membranstruktur einen Rezeptor darstellt für Mannose, IL-1 oder einen Wachstumsfaktor, wie PDGF, FGF, VEGF, TGFβ.

32. Wirkstoff nach Anspruch 30), bei welchem die Membranstruktur Actin, Angiotensin II-Rezeptoren, EGF-Rezeptoren, PDGF-Rezeptoren, FGF-Rezeptoren oder Endothelin-Rezeptoren sind.

33. Wirkstoff nach Anspruch 32., bei welchem die Membranstrukturen Rezeptoren für Zytokine, Wachstumsfaktoren, Interferone, Chemokine darstellen, insbesondere Rezeptoren für den Stem Cell Factor, für II-1, II-2, II-3, II-4, II-6, II-8, II-10, Interferon α, Interferon β, Interferon γ, GM-CSF, M-CSF, G-CSF oder TNFα.

34. Wirkstoff nach den Ansprüchen 1. - 33. in einer Arzneimittelzubereitung für die Injektion in ein Gewebe wie Muskel, Binde-gewebe, Leber, Niere, Milz, Lunge, Haut, für die lokale Applikation auf die Haut oder auf Schleimhäute, für die Injektion in Körperhöhlen wie Gelenke, Pleuralraum, Peritonealraum, Subarachinoidalraum oder für die Injektion in das Blutgefäßsystem wie intraarterielle oder intravenöse Injektion.

## Claims

1. An active compound for the prophylaxis or therapy of diseases of the vascular system, which contains a DNA construct which consists of an activator sequence, a cell cycle-regulated promoter module and a DNA sequence for an active substance which is a cell cycle inhibitor and/or a clotting inhibitor.

2. An active compound as claimed in claim 1., in which the promoter module has the elements CDE-CHR-Inr and contains the positions ≤ -20 to ≥ +30 of the cdc25C promoter range (nucleotide sequence
GGCTGGCGGAAGGTTT-GAATGGTCAACGCCTGCGG-CTGTTGATATTCTTG), where CDE being the "cell cycle dependent element" (nucleotide sequence TGGCGG), CHR the "cell cycle gene homology region" (nucleotide sequence GTTTGAA) and Inr the initiation site (position +1) as well as the adjacent sequences important for initiation.

3. An active compound as claimed in claim 1. and 2. comprising an activator sequence (promoter or enhancer sequence) which is regulated by transcription factors formed in smooth muscle cells.

4. An active compound as claimed in claim 3. comprising
- as activator sequence, the CMV enhancer, the CMV promoter or the SV40 promoter or
- the promoter sequence for the gene of tropomyosin, α-actin, α-myosin, PDGF receptors, FGF receptors, MRF-4, acetylcholine receptors, phosphofructinase A, phosphoglycerate mutase, troponin C, myogenin, endothelin receptors or desmin or
- as activator sequence, multiple copies of a binding site for muscle-specific HLH proteins (E box) or for GATA-4 or
- the promoter sequence for VEGF or the enhancer sequence for VEGF or the DNA sequence for c-Src or for v-Src, which regulate the VEGF gene.

5. An active compound as claimed in claim 1-4., in which the DNA sequence for the active substance encodes a cell cycle inhibitor which is
- the retinoblastoma protein pRb/p110, the p107 protein or the 130 protein or
- the p53 protein or
- the p21 protein, the p16 protein or another "cyclin-dependent kinase (cdk)" inhibitor or
- the GADD45 protein or
- the bak protein or
- a cytostatic or cytotoxic protein or
- an enzyme for the cleavage of precursors of cytostatics to cytostatics.

6. An active compound as claimed in claim 5.,
- in which the retinoblastoma protein (pRb/p110) is no longer phosphorylatable due to replacement of the amino acids in positions 246, 350, 601, 605, 780, 786, 787, 800 and 804, but without losing its binding activity with the large T antigen, for example in that the amino acids Thr-246, Ser-601, Ser-605, Ser-780, Se-786, Ser-787 and Ser-800 are replaced by Ala, the amino acid Thr-350 is replaced by Arg and the amino acid Ser-804 is replaced by Glu or
- in which the p107 protein is mutated in an analogous manner to pRb/110 or
- in which the p130 protein is mutated in an analogous manner to pRb/110.

7. An active compound as claimed in claim 5., in which the protein p53 is C-terminally shortened by serine 392.

8. An active compound as claimed in claim 1-4., in which the cell cycle inhibitor is perforin, granzyme, TNFα or TNFβ.

9. An active compound as claimed in claim 1-4., in which the cell cycle inhibitor is an enzyme and this enzyme is a herpes simplex virus thymidine kinase, cytosine deaminase, varicella zoster virus thymidine kinase, nitroreductase, β-glucuronidase (in particular a human, plant or bacterial β-glucuronidase), carboxypeptidase (preferably of Pseudomonas), lactamase (preferably of Bacillus cereus), pyroglutamate aminopeptidase, D-aminopeptidase, oxidase, peroxidase, phosphatase, hydroxynitrillyase, protease, esterase or a glycosidase, where the signal sequence for this enzyme can be homologous or, for better cellular secretion, also heterologous.

10. An active compound as claimed in claim 9., in which lysosomal storage is decreased by point mutations of the cDNA sequence of the enzyme and the extracellular secretion is increased.

11. An active compound as claimed in claim 1-10., which contains the DNA sequences of a pluarity of identical or different cell cycle inhibitors, two DNA sequences in each case being connected to one another by a DNA sequence for the internal ribosome entry site.

12. An active compound as claimed in claim 1-4., in which the DNA sequence for the active substance encodes a clotting inhibitor.

13. An active compound as claimed in claim 1-10., in which the DNA sequence for a cell cycle inhibitor is supplemented by the DNA sequence for a clotting inhibitor and these two DNA sequences are connected to one another by a DNA sequence for the internal ribosome entry site.

14. An active compound as claimed in claim 12. and 13., in which the DNA sequence for the clotting inhibitor encodes tPA, uPA, hybrid molecules of tPA and uPA, protein C, antithrombin III, TFPI, C-1 inhibitor, al-antitrypsin or hirudine.

15. An active compound as claimed in claim 1. and 2. comprising an activator sequence (promoter or enhancer sequence) which is regulated by transcription factors formed in proliferating endothelial cells.

16. An active compound as claimed in claim 15. comprising
- the promoter sequence for the endothelial glucose-1-transporter, for endoglin, VEGF receptor-1 or -2, receptor tyrosine kinase til-1 or til-2, B61 receptor, B61 ligand, endothelin, in particular endothelin-B, -1, mannose-6-phosphate receptor, Il-1α or Il-1β, I1-1 receptor, VCAM-1, von Willebrand factor or
- a synthetic activator sequence from oligomerized binding sites for transcription factors which are preferentially or selectively active in endothelial cells, such as GATA-2 whose binding site is 5'-TTATCT-3'.

17. An active compound as claimed in claim 1. and 2., comprising an activator sequence (promoter or enhancer sequence) which is regulated by transcription factors which are formed to a particular extent in the activation of and in macrophages or in lymphocytes.

18. An active compound as claimed in claim 17., comprising a promoter sequence for cytokines or their receptors, in particular for interleukin (Il)-1α, or Il-1β, I1-1 receptor, Il-2, Il-2 receptor, interferon γ, Il-3, Il-3 receptor, Il-4, Il-4 receptor, Il-5, Il-6, Il-7, Il-8, Il-9, Il-10, Il-11, Il-12, Il-13, M-CSF receptor, macrophage scavenger type I or type II receptor, interferon regulatory factor 1, IFN-γ responsive promoter, IFN-γ, granulocyte-macrophage colony-stimulating factor (GM-CSF), GM-CSF receptor, granulocyte colony-stimulating factor (G-CSF), adhesion proteins, such as LFA-1, Mac-1 or p150/95, or leukemia-inhibiting factor (LIF).

19. An active compound as claimed in claim 15.-18., in which the DNA sequence for the active substance encodes a clotting inhibitor.

20. An active compound as claimed in claim 19., in which the DNA sequence for the clotting inhibitor encodes tissue plasminogen activator (tPA), urokinase (uPA), hybrids of tPA and uPA, protein C, antithrombin III, the tissue factor pathway inhibitor, C-1 inhibitor, al-antitrypsin or hirudine.

21. An active compound as claimed in claim 1. - 4. and 15. - 20., which contains the DNA sequences of a plurality of identical or different clotting inhibitors, two DNA sequences in each case being connected to one another by a DNA sequence for the internal ribosome entry site.

22. An active compound as claimed in claim 1. - 21., inserted in a vector.

23. An active compound as claimed in claim 22., in which the vector is a virus.

24. An active compound as claimed in claim 22., in which the virus is a retrovirus, adenovirus, adeno-associated virus, herpes simplex virus or vaccinia virus.

25. An active compound as claimed in claim 1. - 24. inserted in a plasmid.

26. An active compound as claimed in claim 22. - 25., prepared in a colloidal dispersion system.

27. An active compound as claimed in claim 26., in which the colloidal dispersion system are liposomes.

28. An active compound as claimed in claim 26., in which the colloidal dispersion system are polylysine ligands.

29. An active compound as claimed in claim 22. - 28. supplemented by a ligand which binds to membrane structures of smooth muscle cells, activated endothelial cells, activated macrophages or activated lymphocytes.

30. An active compound as claimed in claim 22), in which the ligand
- is a polyclonal or monoclonal antibody or an antibody fragment thereof which binds with its variable domains specifically to the membrane structure of smooth muscle cells, activated endothelial cells, activated macrophages or activated lymphocytes or
- is a cytokine or growth factor or a fragment or a partial sequence thereof which binds to receptors on smooth muscle cells, activated endothelial cells, activated macrophages or activated lymphocytes or
- is an adhesion molecule, such as SLeX, LFA-1, MAC-1, LECAM-1 or VLA-4.

31. An active compound as claimed in claim 30), in which the membrane structure is a receptor for mannose, IL-1 or a growth factor, such as PDGF, FGF, VEGF, TGFβ.

32. An active compound as claimed in claim 30), in which the membrane structure are actin, angiotensin II receptors, EGF receptors, PDGF receptors, FGF receptors or endothelin receptors.

33. An active compound as claimed in claim 32., in which the membrane structures are receptors for cytokines, growth factors, interferons, chemokines, in particular receptors for the stem cell factor, for Il-1, Il-2, Il-3, Il-4, Il-6, Il-8, Il-10, interferon α, interferon β, interferon γ, GM-CSF, M-CSF, G-CSF or TNFα.

34. An active compound as claimed in claims 1. - 33. in a pharmaceutical preparation for injection into a tissue such as muscle, connective tissue, liver, kidney, spleen, lung, skin, for local application on the skin or on mucous membranes, for injection into body cavities such as joints, pleural cavity, peritoneal cavity, subarachnoid cavity or for injection into the vascular system such as intra-arterial or intravenous injection.

## Revendications

1. Substance active pour la prophylaxie ou la thérapie de maladies du système vasculaire, caractérisée en ce qu'elle contient un produit de construction de type ADN qui est constitué d'une séquence d'activateur, d'un module promoteur régulé par le cycle cellulaire et d'une séquence d'ADN codant pour une substance active qui représente un inhibiteur de cycle cellulaire et/ou un inhibiteur de coagulation.

2. Substance active selon la revendication 1, dans laquelle le module promoteur possède les éléments CDE-CHR-Inr et contient les positions ≤ -20 à ≥ +30 de la région de promoteur cdc25C (séquence nucléotidique GGCTGGCGGAAGGTTT-GAATGGTCAACGCCTGCGGCTGTTGATATTCTTG), CDE représentant l'élément dépendant du cycle cellulaire *cell cycle dépendent element* (séquence nucléotidique TGGCGG), CHR représentant la région d'homologie de gène de cycle cellulaire *cell cycle gene homology region* (séquence nucléotidique GTTTGAA) et Inr représentant le site d'initiation (position +1) ainsi que les séquences voisines importantes pour l'initiation.

3. Substance active selon les revendications 1 et 2, contenant une séquence d'activateur (séquence de promoteur ou d'activateur) qui est régulée par des facteurs de transcription formés dans des cellules musculaires lisses.

4. Substance active selon la revendication 3, contenant
- en tant que séquence d'activateur, l'activateur de CMV, le promoteur de CMV ou le promoteur de SV40, ou
- la séquence de promoteur pour le gène de la tropomyosine, l'α-actine, l'α-myosine, les récepteurs de PDGF, les récepteurs de FGF, MRF-4, les récepteurs d'acétylcholine, la phosphofructinase A, la phosphoglycérate mutase, la troponine C, la myogénine, les récepteurs d'endothéline ou la desmine, ou
- en tant que séquence d'activateur, des copies multiples d'un site de liaison pour des protéines HLH spécifiques de muscles (boîte E) ou pour GATA-4, ou
- la séquence de promoteur pour VEGF ou la séquence d'activateur pour VEGF, ou la séquence d'ADN codant pour c-Src ou pour v-Src, qui régulent le gène VEGF.

5. Substance active selon les revendications 1 à 4, dans laquelle la séquence d'ADN pour la substance active code pour un inhibiteur de cycle cellulaire qui représente
- la protéine de rétinoblastome pRb/p110, la protéine p107 ou la protéine 130 ou
- la protéine p53 ou
- la protéine p21, la protéine p16 ou un autre inhibiteur de kinase dépendant de la cycline (cdK) ou
- la protéine GADD45 ou
- la protéine bak ou
- une protéine cytostatique ou cytotoxique ou
- une enzyme pour la coupure de précurseurs d'agents cytostatiques en agents cytostatiques.

6. Substance active selon la revendication 5,
- dans laquelle la protéine de rétinoblastome (pRB/p110) n'est plus phosphorylable, par remplacement des aminoacides aux positions 246, 350, 601, 605, 780, 786, 787, 800 et 804, mais sans perdre son activité de liaison avec le grand antigène T, par exemple, par remplacement des aminoacides Thr-246, Ser-601, Ser-605, Ser-780, Ser-786, Ser-787 et Ser-800 par Ala, de l'aminoacide Thr-350 par Arg et de l'aminoacide Ser-804 par Glu, ou
- dans laquelle la protéine p107 est mutée de la même façon que pRb/110, ou
- dans laquelle la protéine p130 est mutée de la même façon que pRb/110.

7. Substance active selon la revendication 5, dans laquelle la protéine p53 est raccourcie de la sérine 392 à l'extrémité C-terminale.

8. Substance active selon les revendications 1 à 4, dans laquelle l'inhibiteur de cycle cellulaire est la perforine, la granzyme, le TNFα ou le TNFβ.

9. Substance active selon les revendications 1 à 4, dans laquelle l'inhibiteur de cycle cellulaire représente une enzyme, et cette enzyme est une thymidine kinase du virus *Herpes simplex,* la cytosine déaminase, la thymidine kinase du virus varicelle-zona, la nitroréductase, la β-glucuronidase (en particulier une β-glucuronidase humaine, végétale ou bactérienne), une carboxypeptidase (de préférence de *Pseudomonas),* une lactamase (de préférence de *Bacillus cereus),* la pyroglutamate aminopeptidase, la D-aminopeptidase, l'oxydase, la peroxydase, la phosphatase, l'hydroxynitrile lyase, une protéase, une estérase ou une glycosidase, la séquence signal pour cette enzyme pouvant être homologue ou bien hétérologue, pour une meilleure excrétion cellulaire.

10. Substance active selon la revendication 9, dans laquelle le stockage lysosomique est diminué et l'excrétion extracellulaire est accrue, par des mutations ponctuelles de la séquence d'ADNc de l'enzyme.

11. Substance active selon les revendications 1 à 10, qui contient les séquences d'ADN de plusieurs inhibiteurs de cycle cellulaire, identiques ou différents, dans chaque cas deux séquences d'ADN pouvant être reliées l'une à l'autre par une séquence d'ADN codant pour le site d'entrée interne du ribosome.

12. Substance active selon les revendications 1 à 4, dans laquelle la séquence d'ADN code pour une substance active d'un inhibiteur de coagulation.

13. Substance active selon les revendications 1 à 10, dans laquelle la séquence d'ADN codant pour un inhibiteur de cycle cellulaire est complétée par la séquence d'ADN codant pour un inhibiteur de coagulation, et ces deux séquences d'ADN sont reliées l'une à l'autre par une séquence d'ADN codant pour le site d'entrée interne du ribosome.

14. Substance active selon les revendications 12 et 13, dans laquelle la séquence d'ADN codant pour l'inhibiteur de coagulation code pour le tPA (activateur tissulaire du plasminogène), l'uPA (activateur du plasminogène de type urokinase), des molécules hybrides de tPA et uPA, la protéine C, l'antithrombine III, le TFPI, l'inhibiteur C-1, l'antitrypsine α1 ou l'hirudine.

15. Substance active selon les revendications 1 et 2, contenant une séquence d'activateur (séquence de promoteur ou d'activateur) qui est régulée par des facteurs de transcription formés dans des cellules endothéliales proliférantes.

16. Substance active selon la revendication 15, contenant
- la séquence de promoteur pour le transporteur endothélial de glucose 1, l'endogline, le récepteur 1 ou 2 de VEGF, le récepteur de tyrosine kinase til-1 ou til-2, le récepteur B61, le ligand B61, une endothéline, en particulier l'endothéline B, le récepteur de mannose-6-phosphate, l'IL-1α ou l'IL-1β, le récepteur d'IL-1, VCAM-1, le facteur von Willebrand ou
- une séquence d'activateur synthétique constituée de sites de liaison oligomérisés pour des facteurs de transcription, qui sont actifs préférentiellement ou sélectivement dans des cellules endothéliales, tels que GATA-2, dont le site de liaison est 5'-TTATCT-3'.

17. Substance active selon les revendications 1 et 2, contenant une séquence d'activateur (séquence de promoteur ou d'activateur) qui est régulée par des facteurs de transcription qui sont formés à un degré particulier lors de l'activation de et dans des macrophages ou dans des lymphocytes.

18. Substance active selon la revendication 17, contenant une séquence de promoteur pour des cytokines ou leurs récepteurs, en particulier pour l'interleukine (IL) 1α ou IL-1β, le récepteur d'IL-1, IL-2, le récepteur d'IL-2, l'interféron γ, IL-3, le récepteur d'IL-3, IL-4, le récepteur d'IL-4, IL-5, IL-6, IL-7, IL-8, IL-9, IL-10, IL-11, IL-12, IL-13, le récepteur de M-CSF, le récepteur de capteur de macrophage de type 1 ou de type 2, le facteur régulateur d'interféron 1, le promoteur répondant à IFNγ, IFNγ, le facteur stimulant la formation de colonies de macrophages-granulocytes (GM-CSF), le récepteur de GM-CSF, le facteur stimulant la formation de colonies de granulocytes (G-CSF), des protéines d'adhésion telles que LFA-1, Mac-1 ou p150/95, ou le facteur inhibiteur de leucémie (LIF).

19. Substance active selon les revendications 15 à 18, dans laquelle la séquence d'ADN codant pour la substance active code pour un inhibiteur de coagulation.

20. Substance active selon la revendication 19, dans laquelle la séquence d'ADN codant pour l'inhibiteur de coagulation code pour l'activateur tissulaire du plasminogène (tPA), l'urokinase (uPA), des hybrides de tPA et uPA, la protéine C, l'antithrombine III, le *Tissue Factor Pathway Inhibitor,* l'inhibiteur C-1, l'antitrypsine α1 ou l'hirudine.

21. Substance active selon les revendications 1 à 4 et 15 à 20, qui contient les séquences d'ADN de plusieurs inhibiteurs de coagulation identiques ou différents, dans chaque cas deux séquences d'ADN étant reliées l'une à l'autre par une séquence d'ADN codant pour le site d'entrée interne du ribosome.

22. Substance active selon les revendications 1 à 21, insérée dans un vecteur.

23. Substance active selon la revendication 22, dans laquelle le vecteur est un virus.

24. Substance active selon la revendication 22, dans laquelle le virus est un rétrovirus, un adénovirus, un virus associé à un adénovirus, le virus *Herpes simplex* ou le virus de la vaccine.

25. Substance active selon les revendications 1 à 24, insérée dans un plasmide.

26. Substance active selon les revendications 22 à 25, préparée dans un système de dispersion colloïdal.

27. Substance active selon la revendication 26, dans laquelle le système de dispersion colloïdal consiste en liposomes.

28. Substance active selon la revendication 26, dans laquelle le système de dispersion colloïdal consiste en ligands de type polylysine.

29. Substance active selon les revendications 22 à 28, complétée par un ligand qui se lie à des structures membranaires de cellules musculaires lisses, de cellules endothéliales activées, de macrophages activés ou de lymphocytes activés.

30. Substance active selon la revendication 22, dans laquelle le ligand
- est un anticorps monoclonal ou polyclonal ou un fragment d'anticorps qui se lie, par ses domaines variables, spécifiquement à la structure membranaire de cellules musculaires lisses, de cellules endothéliales activées, de macrophages activés ou de lymphocytes activés, ou
- est une cytokine ou un facteur de croissance ou un fragment ou une séquence partielle de ceux-ci, qui se lie à des récepteurs sur des cellules musculaires lisses, des cellules endothéliales activées, des macrophages activés ou des lymphocytes activés, ou
- représente une molécule d'adhésion, telle que SLeX, LFA-1, MAC-1, LECAM-1 ou VLA-4.

31. Substance active selon la revendication 30, dans laquelle la structure membranaire représente un récepteur pour le mannose, IL-1 ou un facteur de croissance, tel que PDGF, FGF, VEGF, TGFβ.

32. Substance active selon la revendication 30, dans laquelle la structure membranaire consiste en actine, des récepteurs d'angiotensine II, des récepteurs d'EGF, des récepteurs de PDGF, des récepteurs de FGF ou des récepteurs d'endothéline.

33. Substance active selon la revendication 32, dans laquelle les structures membranaires représentent des récepteurs pour des cytokines, des facteurs de croissance, des interférons, des chémokines, en particulier des récepteurs pour le *Stem Cell Factor,* pour IL-1, IL-2, IL-3, IL-4, IL-6, IL-8, IL-10, l'interféron α, l'interféron β, l'interféron γ, GM-CSF, M-CSF, G-CSF ou TNFα.

34. Substance active selon les revendications 1 à 33, dans une composition médicammenteuse pour l'injection dans un tissu tel que le muscle lisse, le tissu conjonctif, le foie, le rein, la rate, le poumon, la peau, pour l'application locale sur la peau ou les muqueuses, pour l'injection dans des cavités corporelles telles que les articulations, l'espace pleural, l'espace péritonéal, l'espace sous-arachnoïdien ou pour l'injection dans le système vasculaire, telle qu'une injection intraartérielle ou intraveineuse.
